(19) Europäisches Patentamt
European Patent Office
Office européen des brevets

(11) EP 1 008 651 A2

(12) EUROPEAN PATENT APPLICATION

(43) Date of publication:
**14.06.2000 Bulletin 2000/24**

(21) Application number: **99204068.3**

(22) Date of filing: **01.12.1999**

(51) Int. Cl.$^7$: **C12N 15/81**, C12N 15/67, C12N 9/04, C12N 15/53, C12N 1/19 // (C12N1/19, C12R1:84)

(84) Designated Contracting States:
**AT BE CH CY DE DK ES FI FR GB GR IE IT LI LU MC NL PT SE**
Designated Extension States:
**AL LT LV MK RO SI**

(30) Priority: **09.12.1998 DK 163098**

(71) Applicant:
**Bioteknologisk Institut**
**2970 Hoersholm (DK)**

(72) Inventors:
- **Stougaard, Peter**
  **4050 Skibby (DK)**
- **Pedersen, Lars H.**
  **2750 Ballerup (DK)**
- **Wolff, Anne Mette**
  **2820 Gentofte (DK)**
- **Poulsen, Ulla**
  **2830 Virum (DK)**
- **Hansen, Ole C.**
  **2100 Copenhagen OE (DK)**

(74) Representative:
**Plougmann, Vingtoft & Partners A/S**
**Sankt Annae Plads 11,**
**P.O. Box 3007**
**1021 Copenhagen K (DK)**

(54) **Modified DNA sequence coding for hexose oxidase and use hereof**

(57) A nucleic acid fragment comprising a modified nucleotide sequence coding for a hexose oxidase that is obtainable by selecting a source organism naturally producing a hexose oxidase including algal species such as *Chondrus crispus*, *Iridophycus flaccidum* and *Euthora cristata*, isolating from said organism the nucleotide sequence coding for hexose oxidase and modifying the sequence e.g. by substituting one or more codons with codons which are used by the selected host organism. The modified nucleotide sequence is expressible in a host organism at a level which, relative to the level at which the non-modified nucleotide sequence is expressed in the host organism under substantially identical conditions, is at least 10% higher. The nucleic acid fragment is useful as a means of recombinantly producing hexose oxidase in prokaryotic and eukaryotic host cells including bacterial, yeast and fungal species such as the bacterial species *E. coli* and the yeast species *Pichia pastoris* and *Saccharomyces* species.

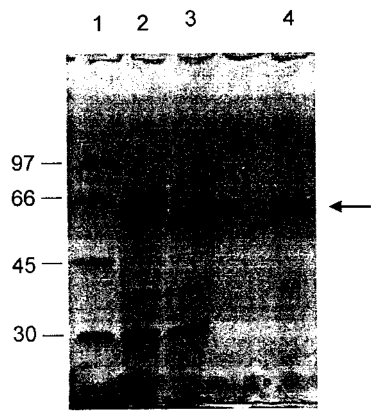


Fig. 1

EP 1 008 651 A2

## Description

FIELD OF THE INVENTION

The present invention relates to the field of recombinant DNA technology for production of industrially useful enzyme products. In particular, there is provided an improved method of producing hexose oxidase and more specifically, the invention provides a modified DNA sequence coding for hexose oxidase which, relative to the non-modified sequence from which it is derived, results in an enhanced expression of the enzyme.

TECHNICAL BACKGROUND AND PRIOR ART

Hexose oxidase (D-hexose: $O_2$-oxidoreductase, EC 1.1.3.5) is an enzyme that in the presence of oxygen is capable of oxidising D-glucose and several other reducing sugars including maltose, lactose and cellobiose to their corresponding lactones with subsequent hydrolysis to the respective aldibionic acids. Accordingly, hexose oxidase differs from another oxidoreductase, glucose oxidase, which can only convert D-glucose, in that the enzyme can utilise a broader range of sugar substrates. The oxidation catalysed by hexose oxidase can be illustrated as follows:

$$\text{D-glucose} + O_2 \rightarrow \gamma\text{-D-gluconolactone} + H_2O_2$$

, or

$$\text{D-galactose} + O_2 \rightarrow \gamma\text{-D-galactonolactone} + H_2O_2$$

Hexose oxidase (in the following also referred to as HOX) is produced naturally by several marine algal species. Such species are i.a. found in the family *Gigartinaceae* that belongs the order *Gigartinales*. Examples of hexose oxidase producing algal species belonging to *Gigartinaceae* include *Chondrus crispus* and *Iridophycus flaccidum*. Also algal species of the order *Cryptomeniales* including the species *Euthora cristata* are potential natural sources of hexose oxidase.

Until recently, the only means of providing hexose oxidase has been to isolate the enzyme from a source organism that naturally produces the enzyme. Co-pending international patent application, WO 96/40935 discloses the first successful cloning and expression in recombinant host organisms of a gene derived from a marine algal species and coding for hexose oxidase. Thus, it was found that such a gene having a coding DNA sequence of the source organism could be expressed in both prokaryotic and eukaryotic micro-organisms including *Escherichia coli*, *Pichia pastoris* and *Saccharomyces cerevisiae*. It has subsequently been found, however, that the level of expression in such recombinant host organisms may be relatively low implying that an industrially feasible production of the enzyme is not attainable.

It is therefore a major objective of the present invention to provide an improved method of producing hexose oxidase by means of a recombinant host organism such as *Pichia pastoris*. In particular, it was an objective to obtain a fermentation process where the expression level of the enzyme is at least 250 mg per litre of fermentation medium.

The yeast *Pichia pastoris* is a widely used host organism for the expression of heterologous genes including a relatively limited number of plant genes (Cregg et al., 1993; Shu-Hsing et al., 1996). Although relatively high levels of expression of heterologous genes in this organism have been reported, it is also known that several factors may adversely affect the usefulness of *Pichia pastoris* expression systems.

One such factor is the inherent proteolytic activity of this organism (Sreekrishna et al. 1996; Cregg et al., 1993) causing significant degradation of the gene products of interest. Another factor of significance for the achievement of attractive levels of gene expression is the nucleotide sequence of the foreign gene to be expressed. Thus, it has been found that genes with high A+T content are not transcribed efficiently in *Pichia pastoris* due to premature termination and it was suggested by Sreekrishna et al. (1997) that, as a general strategy to overcome this problem, the genes to be expressed should be redesigned so as to have an A+T content in the range of 30-55%. An additional factor which may cause inefficient expression of cysteine rich proteins is that they are expressed as incorrectly disulphide-bonded aggregates, and hence in inactive form (http://www.invitrogen.com/bin/wwwboard/pichia/messages/90.html).

Sreekrishna et al. (1996) have reviewed strategies for optimising the synthesis and secretion of heterologous proteins in *Pichia pastoris*. Such strategies include the use of integrative plasmids, choice of site of integration of the expression vector, increasing the gene dosage, optimising the nucleotide sequence and the length of the 5'-*UTR*, modifying the initial portion of the coding sequences with alternate codons, redesigning the gene so as to have an A+T content in the range of 30-55% and improving the proteolytic stability of secreted proteins. In that context, these authors indicated that for designing synthetic genes, use of *Pichia pastoris* preferred codons is recommended.

The present invention relates specifically to the expression of the algal protein hexose oxidase in *Pichia*

*pastoris*. The naturally occurring hexose oxidase gene from *Chondrus crispus* has an A+T content of 42%, i.e. within the range which according to the literature should not have an adverse effect on the expression of the gene in *Pichia pastoris*. It would therefore appear *a priori* that differences in codon usage between the algal species and *Pichia pastoris* was not the cause of low expression of the hexose oxidase gene.

However, faced with the problem that the expression in *Pichia pastoris* of a wild type plant derived hexose oxidase gene had not been at a commercially attractive level, the present inventors nevertheless chose to modify the nucleotide sequence of the hexose oxidase coding sequence to substantially bring it into accordance with preferred codon usage in *Pichia pastoris* and not, as would have been more obvious, to use any of the other means of optimising gene expression that were offered in the literature. Having made this modification of the coding sequence, it appeared, however, that the A+T content of the thus modified gene had increased from 42 to 53%, i.e. close to the upper limit of the range indicated in the literature as being useful. It was therefore a highly unexpected finding that the expression level increased to the extent achieved in the invention.

Additionally, it is known that major differences in codon usage between a foreign gene and wild type organisms such as *E. coli* may adversely affect efficient expression of the foreign gene in that organism (Makrides, 1996). However, this author reported that several workers have found very efficient expression of genes that contained low-usage codons and it was stated that to date it had not been possible to formulate general and unambiguous "rules" to predict whether the content of low-usage codons in a specific gene might adversely affect the efficiency of its expression in and that experimental results may be confounded by several variables, such as positional effects, the clustering or interdispersion of the rarely used codons, the secondary structure of the mRNA, and other effects. The implication of this is that it cannot be predicted whether the replacement of low usage codons with preferred codons will result in an enhancement of expression. In Makrides (1996) it is thus i.a. concluded that currently, all the rules that link codon usage and translation of a transcript are not known. This is illlustrated clearly by Hoekema et al. (1987) who studied the role of biased codon usage in gene expression in the yeast *Saccharomyces cerevisiae*. Among the findings of these authors was that the introduction of 22 consecutive rare codons at the 5' end of the PGK gene in the plasmid YEpR7 caused no significant difference in expression level.

It could therefore not be predicted that a modification of the wild type gene coding for hexose oxidase to correspond to codon usage in *Pichia pastoris* would result in an enhancement of the expression level.

## SUMMARY OF THE INVENTION

Accordingly, the invention pertains in a first aspect to a nucleic acid fragment comprising a modified nucleotide sequence coding for a hexose oxidase, the fragment is obtainable by selecting a source organism naturally producing a hexose oxidase, isolating from said organism the nucleotide sequence coding for said naturally produced hexose oxidase and modifying said sequence, the thus modified nucleotide sequence is expressible in a host organism at a level which, relative to the level at which the non-modified nucleotide sequence is expressed in said host organism under substantially identical conditions, is at least 10% higher.

In a further aspect, the invention relates to a method of preparing a nucleic acid fragment comprising a modified nucleotide sequence coding for a hexose oxidase, the method comprising the step of (i) selecting a source organism naturally producing a hexose oxidase, (ii) isolating from said source organism a nucleic acid fragment comprising the nucleotide sequence coding for said naturally produced hexose oxidase, (iii) selecting a host organism and determining for said host organism the frequency of nucleotide triplets (codons) coding for individual amino acids (codon usage), and (iv) modifying the isolated nucleotide sequence by substituting at least one codon coding for an amino acid with a different codon coding for the same amino acid, said different codon is used naturally by said host organism at a higher frequency than the codon being substituted, and (v) selecting a thus modified nucleotide sequence that, when expressed in the selected host organism, is expressible in the selected host organism at a level which, relative to the level at which the non-modified nucleotide sequence is expressed in said host organism under substantially identical conditions, is at least 10% higher.

In still further aspects of the invention there is provided an expression vector that comprises the above nucleic acid fragment and a host organism that is transformed with such an expression vector.

In other aspects the invention relates to a method of producing hexose oxidase, the method comprising the steps of (i) cultivating a host organism as defined above in an appropriate medium under conditions where the nucleotide sequence coding for the hexose oxidase is expressed, and (ii) harvesting the hexose oxidase, and a method of producing a polypeptide having hexose oxidase activity, the method comprising isolating a DNA fragment comprising a sequence coding for the polypeptide, transforming a host organism with said coding sequence, cultivating the thus transformed host organism under conditions leading to expression of the hexose oxidase active polypeptide and recovering the polypeptide from the cultivation medium, the polypeptide being expressed at an amount that is at least 100 mg per litre of the culture medium.

DETAILED DISCLOSURE OF THE INVENTION

It is a major objective of the invention to provide a nucleic acid fragment comprising a modified nucleotide sequence coding for a hexose oxidase that is expressible in a host organism at a level which, relative to the level at which the corresponding non-modified nucleotide sequence is expressed in said host organism under substantially identical conditions, is at least 10% higher such as at least 50% higher including at least 100% higher.

Hexose oxidase is produced naturally by several marine algal species. Such species are i.a. found in the family *Gigartinaceae* that belongs to the order *Gigartinales*. Examples of hexose oxidase producing algal species belonging to *Gigartinaceae* include *Chondrus crispus* and *Iridophycus flaccidum*. Also algal species of the order *Cryptomeniales* including the species *Euthora cristata* are potential sources of the hexose oxidase. Accordingly, any of the above algal species are potentially useful sources of nucleotide sequences coding for hexose oxidase. As used herein, the term "hexose oxidase" denotes an enzyme which at least oxidises D-glucose, D-galactose, D-mannose, maltose, lactose and cellobiose.

In accordance with the invention, the nucleotide sequence coding for hexose oxidase can be obtained by selecting a source organism naturally producing hexose oxidase including any of the above algal species or other plant species and a micro-organism, isolating or deriving from said organism the wild type nucleotide sequence coding for the naturally produced hexose oxidase and modifying the coding sequence so as to obtain a coding sequence that is expressed at a higher level in the selected host organism than is the wild type sequence. The isolation of the sequence can be carried out as disclosed in the copending application WO 96/40935 or it can be derived by constructing it synthetically by established standard methods as it is also described in WO 96/40935.

The above method implies that the modified coding sequence is introduced into the selected host organism in which the sequence is expressible. Accordingly, the modified coding sequence is combined operably with one or more appropriate expression signal(s) for the coding sequence. Such an introduction can be carried out by methods that are well known to the skilled practitioner including the construction of a vector having the sequence inserted and transforming the host organism with the vector. Suitable vectors include plasmids that are capable of replication in the selected host organism. It is also conceivable that the coding sequence can be integrated in the chromosome of the host organism e.g. by inserting the sequence into a transposable element such as a transposon, and subjecting the mixture of the selected host organism and the transposon to conditions where the transposon will become integrated into the host organism chromosome and combine with the appropriate expression signal.

It will be understood that in order to select a modified sequence that is expressed in the selected host organism at the above desired level it may be required to construct, starting from the wild type sequence, a multiplicity of differently modified sequences or to construct, starting from a previously modified sequence, one or more further modified sequences and to test such modified sequences in the host organism and select one or more transformant strains that has the desired expression level.

In accordance with the invention, a suitable host organism includes a cell of a higher organism such as an animal cell, including a mammal, an avian or an insect cell, or a plant cell. In preferred embodiments, the host organism is a microbial cell, e.g. a bacterial cell or a fungal cell including a yeast cell.

Examples of suitable bacterial host organisms are gram positive bacterial species such as *Bacillaceae* including *Bacillus subtilis*, *Bacillus licheniformis*, *Bacillus lentus*, *Bacillus brevis*, *Bacillus stearothermophilus*, *Bacillus alkalophilus*, *Bacillus amyloliquefaciens*, *Bacillus coagulans*, *Bacillus lautus*, *Bacillus megaterium* and *Bacillus thuringiensis*, *Streptomyces* species such as *Streptomyces murinus*, lactic acid bacterial species including *Lactococcus* spp. such as *Lactococcus lactis*, *Lactobacillus* spp. including *Lactobacillus reuteri*, *Leuconostoc* spp., *Pediococcus* spp. and *Streptococcus* spp. Alternatively, strains of a gram-negative bacterial species belonging to *Enterobacteriaceae* including *E. coli*, or to *Pseudomonadaceae* can be selected as the host organism.

A suitable yeast host organism can be selected from a species of *Saccharomyces* including *Saccharomyces cerevisiae* or a species belonging to *Schizosaccharomyces*. Suitable host organisms among filamentous fungi include species of *Aspergillus*, e.g. *Aspergillus niger*, *Aspergillus oryzae*, *Aspergillus tubigensis* or *Aspergillus nidulans*. Alternatively, strains of a *Fusarium* species, e.g. *Fusarium oxysporum* or of a *Rhizomucor* species such as *Rhizomucor miehei* can be used as the host organism.

In one presently preferred embodiment, a strain of the methylotrophic yeast species *Pichia pastoris* is used as the host organism.

In one presently preferred embodiment, the nucleic acid fragment comprising the hexose oxidase coding nucleotide sequence is obtained by (i) selecting a source organism naturally producing a hexose oxidase, isolating or deriving from that organism the wild type nucleotide sequence coding for the naturally produced hexose oxidase as it is described above and determining its nucleotide sequence, (ii) selecting a host organism, (iii) determining for that host organism the frequency of nucleotide triplets (codons) coding for individual amino acids (codon usage), and (iv) modifying the isolated nucleotide sequence by substituting at least one codon coding for an amino acid with a different codon coding for the same amino acid including such a different codon that is used naturally by said host organism at a higher frequency than the codon being substituted.

The number of codons that it required to substitute to achieve the desired expression level will depend on the type of the selected host organism and the nucleotide sequence of the wild type coding sequence. Thus, in suitable embodiments at least 5% of the wild type codons are substituted by codons used more frequently by the selected host organism, including at least 10% of wild type codons such at least 20% of wild type codons, e.g. at least 50% of the wild type codons. It will be appreciated that the substitutions can be introduced into the N-terminal half and/or the C-terminal half of the starting coding sequence. The substitutions can be carried out using conventional methods for nucleotide substitutions, e.g. by means of PCR methods as it is described in details in the below examples. Alternatively, a modified sequence can be constructed synthetically.

It is within the scope of the invention that the coding sequence that is modified in accordance with the invention is further modified to comprise at least one codon that codes for an amino acid residue which is not coded for by the wild type sequence coding for the naturally produced hexose oxidase. Such a modification is typically made by conventional methods such as PCR. Thus, it is possible to modify the sequence so as to encode a hexose oxidase comprising, relative to the wild type enzyme, at least two different amino acid residues, such as at least five different amino acid residues including at least ten different amino acid residues.

One example of a suitable wild type nucleotide sequence coding for a naturally produced hexose oxidase that can be used as a starting material for providing a modified coding sequence according to the invention is SEQ ID NO:30 as shown in WO 96/40935. Examples of a nucleic acid fragment comprising a hexose oxidase coding nucleotide sequence that is modified in accordance with the invention include the sequence shown herein as SEQ ID NO:30 and a nucleic acid fragment according to the invention that is derived from that sequence by a modification whereby the coding sequence has at least one codon coding for an amino acid residue that is not encoded by SEQ ID NO:30.

In one useful embodiment, the nucleic acid fragment according to the invention comprises a coding sequence for hexose oxidase that is extended by at least one codon at one end or at both ends. Thus, such an extension may e.g. comprise 1-20 including 2-15 such as 3-10 codons immediately upstream of the translation initiation codon including as an example codons that code for the amino acid sequences Met-Asp-Thr-Arg-Ser- or Met-Glu-Ala-Glu-Ala-. It will be appreciated that such an extension of the coding sequence will result in the expression of an N-terminally extended hexose oxidase polypeptide. In another example, the coding sequence is extended by 1-20 including 2-15 such as 3-10 codons at the 3' end such as e.g by codons coding for the amino acid sequence -Ser-Lys-$Leu_{COOH}$. It will be appreciated that such an extension will result in the expression of a C-terminally extended hexose oxidase.

The provision of such extended coding sequences permits the expression of a tagged hexose oxidase polypeptide where the tags may serve the purpose of facilitating the isolation of the expressed enzyme e.g. by affinity chromatography where the tags bind to the affinity chromatography matrix material. Accordingly, the composition of the tags is preferably selected so as to provide tags having a strong affinity to the selected matrix material.

In a further aspect, the invention pertains to a method of preparing a nucleic acid fragment comprising a modified nucleotide sequence coding for a hexose oxidase, the method comprising the step of (i) selecting a source organism naturally producing a hexose oxidase, (ii) isolating or deriving from said source organism a nucleic acid fragment comprising the nucleotide sequence coding for said naturally produced hexose oxidase, (iii) selecting a host organism and determining for said host organism the frequency of nucleotide triplets (codons) coding for individual amino acids (codon usage), (iv) modifying the isolated nucleotide sequence by substituting at least one codon coding for an amino acid with a different codon coding for the same amino acid including such a different codon that is used naturally by said host organism at a higher frequency than the codon being substituted, and (v) selecting a thus modified nucleotide sequence that, when expressed in the selected host organism, is expressible in the selected host organism at a level which, relative to the level at which the non-modified nucleotide sequence is expressed in said host organism under substantially identical conditions, is at least 10% higher.

The steps of this method are carried out as described above. Thus, the starting nucleic acid fragment comprising the nucleotide sequence coding for the naturally produced hexose oxidase can be isolated from any of the above algal species including a species selected from *Chondrus crispus*, *Iridophycus flaccidum* and *Euthora cristata* or another plant species, or from a microorganism that naturally produces hexose oxidase.

One example of a suitable wild type nucleotide sequence coding for a naturally produced hexose oxidase that can be used as a starting material for providing a modified coding sequence according to the invention is SEQ ID NO:30 as shown in WO 96/40935. Examples of a nucleic acid fragment comprising a hexose oxidase coding nucleotide sequence that is modified in accordance with the method include the sequence shown herein as SEQ ID NO:30 or part hereof and a nucleic acid fragment according to the invention that is derived from that sequence by a modification whereby the coding sequence has at least one codon coding for an amino acid residue that is not encoded by SEQ ID NO:30

In accordance with the invention, a hexose oxidase coding nucleotide sequence that is modified as described herein can be expressed at the desired levels using an expression vector. It is therefore another object of the invention to provide an expression vector comprising a nucleic acid fragment as described above. The expression vector typically includes the components of a cloning vector, i.e. an element that permits autonomous replication of the vector in the selected host organism and one or more phenotypically detectable markers for selection purposes. The expression vector normally comprises control nucleotide sequences encoding a promoter, operator, ribosome binding site, translation initiation signal and optionally, a repressor gene or one or more activator genes. Additionally, the expression vector may comprise a sequence coding for an amino acid sequence capable of targeting the hexose oxidase to a host cell organel such as a peroxisome or to a particular host cell compartment. Such a targeting sequence includes as one example the sequence SKL. To permit the secretion of the expressed enzyme, a secretion signal or a signal peptide may be inserted upstream of the modified coding sequence. In the present context, the term "expression signal" includes any of the above control sequences, repressor or activator sequences and signal sequence. For expression under the direction of control sequences, the hexose oxidase coding sequence is operably linked to the control sequences in proper manner with respect to expression. Promoter sequences that can be incorporated into plasmid vectors, and which can support the transcription of the modified hexose oxidase coding nucleotide sequence include, but are not limited to the *Pichia pastoris AOX*1 or *AOX*2 promoter, the *tac* promoter, phage lambda-derived promoters including the $P_L$ and $P_R$ promoters.

An expression vector carrying the modified nucleotide sequence according to the invention can be any vector which is capable of expressing the hexose oxidase gene in the selected host organism, and the choice of vector will depend on the host cell into which it is to be introduced. Thus, the vector can be an autonomously replicating vector, i.e. a vector that exists as an episomal entity, the replication of which is independent of chromosomal replication, e.g. a plasmid, a bacteriophage or an episomal element, a minichromosome or an artificial chromosome. Alternatively, the vector according to the invention is one which, when introduced into a host cell, is integrated into the host cell genome and replicated together with the chromosome.

In the vector, the nucleotide sequence coding for the hexose oxidase is operably combined with a suitable promoter sequence. The promoter can be any DNA sequence having transcription activity in the host organism of choice and can be derived from genes that are homologous or heterologous to the host organism. Examples of suitable promoters for directing the transcription of the modified nucleotide sequence of the invention in a bacterial host include the promoter of the *lac* operon of *E. coli*, the *Streptomyces coelicolor* agarase gene *dagA* promoters, the promoters of the *Bacillus licheniformis* α-amylase gene (*amyL*), the promoters of the *Bacillus stearothermophilus* maltogenic amylase gene (*amyM*), the promoters of the Bacillus *amyloliquefaciens* α-amylase gene (*amyQ*), the promoters of the *Bacillus subtilis xylA* and *xylB* genes and a promoter derived from a *Lactococcus* sp.-derived promoter including the P170 promoter.

For transcription in a fungal species, examples of useful promoters are those derived form the genes encoding the *Pichia pastoris* alcohol oxidases, *Aspergillus oryzae* TAKA amylase, *Rhizomucor miehei* aspartic proteinase, *Aspergillus niger* neutral α-amylase, *A. niger* acid stable α-amylase, *A. niger* glucoamylase, *Rhizomucor miehei* lipase, *Aspergillus oryzae* alkaline protease, *Aspergillus oryzae* triose phosphate isomerase or *Aspergillus nidulans* acetamidase. As examples of suitable promoters for the expression in a yeast species, the Gal 1 and Gal 10 promoters of *Saccharomyces cerevisiae* can be mentioned. When expressed in a bacterial species such as *E. coli*, a suitable promoter can e.g. be selected from a bacteriophage promoter including a T7 promoter and a phage lambda promoter.

During the experimentation leading to the present invention it was found that the nucleotide sequence immediately upstream of the translation initiation codon of the modified nucleotide sequence coding for hexose oxidase has a significant effect on the expression level of the modified sequence. Accordingly, in specific embodiments, the expression vector comprises a nucleic acid fragment comprising, upstream of the translation initiation codon of the modified nucleotide sequence coding for hexose oxidase, a nucleotide sequence that has an enhancing effect on the expression of the modified sequence including a sequence selected from TTATTCGAAGC and GGATCCAAACC.

In other specific embodiment, the expression vector is selected from the plasmids pUPO340 (DSM 12535), pUPO346 (DSM 12530) and pUPO349 (DSM 12531).

In a still further aspect, the invention provides a host organism that is transformed with the above vector. In accordance with the invention the host organism is selected from a bacterial species, a fungal species, a plant cell and an animal cell including any of the host organisms mentioned above. A presently preferred host organism is a yeast organism including *Pichia pastoris* or a *Saccharomyces* species.

Several of the above host organisms such as fungal species or gram positive bacterial species can be transformed with the vector by a process that includes protoplast formation and transformation of the protoplasts followed by regeneration of the cell wall in a manner known *per se*.

In advantageous embodiments, the host organism of the invention comprises a DNA sequence coding for a signal peptide that permits the hexose oxidase to be translocated across an intracellular compartment membrane and/or the host organism cell membrane. In other useful embodiments, the host organism is one where the nucleotide sequence coding for hexose oxidase is under the control of a regulatory nucleotide sequence not naturally associated with the coding sequence.

It is also an objective of the invention to provide a method of producing hexose oxidase, comprising the steps of (i) cultivating the host organism according to the invention in an appropriate medium under conditions where the modified nucleotide sequence coding for the hexose oxidase is expressed in a recoverable form, and (ii) harvesting the hexose oxidase according to any conventional method of recovering proteins. The medium used to cultivate the host organism cells can be any conventional medium suitable for cultivating the selected host organism and obtaining expression of the enzyme. Suitable media are available from commercial suppliers or they can be prepared according to recipes that are known to the skilled artisan. It will be appreciated that the above production method includes any production carried out at an industrial scale in suitable production facilities such as fermentors having a volume in the range of 10 to 50,000 litres.

It is a major objective of the invention to provide a method of producing hexose oxidase at high efficiencies. Thus, in preferred embodiments, the amount of hexose oxidase that is expressed is at least 250 mg per liter of the total cell culture volume after cultivation of the host organism. In more preferred embodiments, the amount of enzyme that is expressed is at least 500 mg per litre, such as at least 1 g per litre or even at least 2 g per litre of culture volume.

The production method of the invention may further comprise a step of subjecting the harvested hexose oxidase to at least one further purification step.

In another aspect of the invention there is provided a method of producing a polypeptide having hexose oxidase activity, comprising isolating a DNA fragment comprising a sequence coding for the polypeptide, transforming a host organism with said coding sequence, cultivating the thus transformed host organism under conditions leading to expression of the hexose oxidase active polypeptide and recovering the polypeptide from the cell culture volume, the polypeptide being expressed at an amount that is at least 100 mg per litre of the culture volume such as at least 250 mg per litre, including at least 500 mg per litre.

The invention is further illustrated in the below non-limiting examples and the drawings wherein

Fig. 1 shows SDS-PAGE of recombinant hexose oxidase produced in *Escherichia coli*. Lane 1 shows a molecular weight standard (x $10^{-3}$). Lanes 2 and 3 show a crude extract from *E. coli* with pUPO181 induced to express hexose oxidase. Lane 4 shows hexose oxidase purified by preparative SDS-PAGE and electroelution. Arrow shows the position of the hexose oxidase band;

Fig. 2 shows the construction of plasmid pUPO314. Three parts of the hexose oxidase gene were synthesised, the ends of the DNA fragments were trimmed with restriction endonucleases and the parts were ligated into the vector pSCREEN. Grey boxes indicate the hexose oxidase gene, the small white circle indicates a $Lys_{332}$ to $Pro_{332}$ mutation;

Fig. 3 shows the construction of plasmid pUPO316, in which the $Pro_{332}$ mutation has been corrected to the wild type $Lys_{332}$. A synthetic 106 bp oligonucleotide was ligated to the two parts of the synthetic hexose oxidase gene as indicated in the figure, resulting in plasmid pUPO316 (grey boxes indicate the hexose oxidase gene, the small white circle indicate the $Lys_{332}$ to $Pro_{332}$ mutation);

Fig. 4 shows construction of *Pichia pastoris* vectors expressing hexose oxidase. The synthetic hexose oxidase gene with the $Lys_{332}$ to $Pro_{332}$ mutation (from pUPO314) was cloned into the *P. pastoris* expression vector pPIC3 in two ways, giving rise to plasmids pUPO340 and pUPO346. A hexose oxidase gene with the wild type $Lys_{332}$ (from pUPO316) was similarly inserted into pPIC3 resulting in pUPO349. Plasmids pUPO340 and pUPO346 are similar, apart from the DNA context just upstream of the hexose oxidase coding region. Plasmids pUPO346 and pUPO349 are similar, apart from the amino acids $Pro_{332}$ and $Lys_{332}$, respectively. (Grey boxes indicate the hexose oxidase gene, the small white circle indicate the $Lys_{332}$ to $Pro_{332}$ mutation);

Fig. 5 shows a Western blot of recombinant hexose oxidase in intracellular extracts of *Pichia pastoris*. Lane 1 shows molecular weight standard (x $10^{-3}$). Lanes 2, 4, and 6 show hexose oxidase expressed from the plasmid pUPO153 (native, algal codon usage). Lanes 3, 5, and 7 show hexose oxidase from the vector pUPO340 (synthetic gene). *P. pastoris* cells expressing hexose oxidase were analysed on day 1 (lanes 2 and 3), on day 2 (lanes 4 and 5), and on day 3 (lanes 6 and 7) after induction;

Fig. 6 shows a Western blot of recombinant hexose oxidase in intracellular extracts of *Pichia pastoris* two days after induction. Lane 1 shows a molecular weight standard (x $10^{-3}$). Lane 2 shows the presence of recombinant hexose oxidase in extracts of *P. pastoris* cells harbouring plasmid pUPO153 (native, algal codon usage). Lanes 3, 4, and 5 show the presence of hexose oxidase in extracts of cells containing pUPO340, pUPO 346, and pUPO 349, respectively (all synthetic gene constructs);

Fig. 7 shows SDS-PAGE of recombinant hexose oxidase produced in *Pichia pastoris*. Lane 1 shows a molecular weight standard (x $10^{-3}$), lane 2 shows an intracellular extract corresponding to 2 µl of cell culture and in lane 3 is shown a TCA precipitated sample corresponding to a volume of 100 µl extracellular medium. All lanes were run on the same gel that was stained with Comassie Brilliant Blue R-250;

Figs. 8a and 8b show the construction of plasmids pUPO453 and pUPO455 encoding N-terminally extended hexose oxidase. Grey boxes indicate the hexose oxidase gene, the small white circle in pUPO340 indicate the $Lys_{332}$ to $Pro_{332}$ mutation. The black boxes indicate synthetic DNA encoding the extra N-terminal amino acids, shown in their IUPAC one letter code;

Fig. 9 shows production (A) and activity (B) of N- and C-terminally extended hexose oxidase in intracellular extracts of *Pichia pastoris* four days after induction. In A is shown a Western blot analysis of extracts from *Pichia pastoris* containing the wild type hexose oxidase (lane 1), N-terminally extended hexose oxidase variants M-D-T-R-S- (lane 2) and M-E-A-E-A- (lane 3) and the C-terminally extended -S-K-$L_{COOH}$ (lane 4). In B is shown the corresponding hexose oxidase enzyme activity of the extracts analysed in the Western blot analysis (A). Ordinates show the relative hexose oxidase activity in arbitrary units. Lanes as in A; and

Figure 10 shows the construction of plasmid pUPO387 encoding C-terminally extended hexose oxidase. Grey and black boxes indicate hexose oxidase gene and synthetic DNA encoding the extra C-terminal amino acids, respectively. The amino acids are shown according to the IUPAC one letter code.

EXAMPLE 1

**Production of hexose oxidase specific antibodies**

Recombinant hexose oxidase was produced in *Escherichia coli* from the expression plasmid pUPO181 as described in the co-pending application WO 96/40935. The crude extract of *E. coli* cells expressing recombinant hexose oxidase was analysed by SDS-PAGE. A prominent protein band at the relative molecular weight (Mr) 62 kD corresponding to hexose oxidase was transferred to a polyvinylidene difluoride (PVDF) membrane and subjected to N-terminal amino acid sequence analysis as described in WO 96/40935. The amino acid sequence identified was: Ala-Thr-Leu-Pro-Gln-Lys-Asp-Pro-Gly-Tyr- (SEQ ID NO:1). This sequence corresponded to amino acids Nos. 2-11 in the published sequence for hexose oxidase (Hansen and Stougaard, 1997). Therefore, it was concluded that the expressed 62 kD protein was recombinant hexose oxidase lacking the N-terminal amino acid methionine, $Met_1$.

The 62 kD hexose oxidase band observed in SDS-PAGE was purified by preparative SDS-PAGE and electroelution from the gel as described by Hunkapiller et al. (1983). The purity of the electroeluted 62 kD hexose oxidase band was analysed by SDS-PAGE (Fig. 1, lane 4) and by amino acid sequence analysis as described above. The purified hexose oxidase was used for antibody production in rabbits. Portions of approximately 50 µg were mixed with an equal volume of incomplete Freund's adjuvant and used for immunization.

The hexose oxidase specific polyclonal antibodies that were produced in the rabbits were used throughout this study in Western blot analyses. Proteins to be analysed by Western blot analysis were electrophoresed as described above and transferred to a nitrocellulose filter according to standard procedures. The nitrocellulose membrane was blocked 1 hour in a TBS-T solution (50 mM Tris, pH 7.5; 150 mM NaCl; 0,1 % Tween-20) containing 3% skimmed milk powder. Hexose oxidase specific antibodies diluted 1:10,000 in TBS-T containing 1.5% skimmed milk powder were added and the blot was incubated over night. The blot was washed three times in TBS-T before incubation (1 to 2 hours) with the secondary antibody (alkaline phosphatase-conjugated goat anti-rabbit immunoglobulins (DAKO, cat. no. D0487), diluted 1:1000 in TBS-T containing 1.5% skimmed milk powder. The blot was subsequently washed in TBS-T (2 x 20 min) and in TBS (50 mM Tris, pH 7.5; 150 mM NaCl; 1 x 5 min) before development in Nitroblue tetrazolium/5-Bromo-4-chloro-3-indolylphosphate (NBT/BCIP) buffer according to standard procedures.

The specificity of the antibodies was investigated in a series of Western blots with hexose oxidase containing extracts from *Chondrus crispus*, *E. coli* and *Pichia pastoris*, respectively. Western blot analysis of hexose oxidase containing extracts of *P. pastoris* showed a strong hexose oxidase specific band at Mr 62 kD in addition to two or three weaker bands at lower molecular weight (Figs. 5-8).

EXAMPLE 2

**Construction of a synthetic hexose oxidase gene**

A comparison of the codon usage in the native hexose oxidase gene from the red algal species *C. crispus* and that of highly expressed native *P. pastoris* genes showed that several of the codons in the native gene occurred at low frequencies or were absent in highly expressed *P. pastoris* genes (Sreekrishna, 1996; Hansen and Stougaard, 1997; Provart, 1997). Therefore, twenty synthetic oligonucleotides (Table 1), designated hox 1-20, were designed in order to synthesize a hexose oxidase gene with a codon usage possibly optimized for expression in *P. pastoris*.

Table 1. Synthetic oligonucleotide primers used in synthesis of hexose oxidase genes with a codon usage optimized for the production in *Pichia pastoris* (SEQ ID NO: 2-29).

| Primer | DNA sequence | |
|---|---|---|
| hox1 | ACTCCATGGCTACTTTGCCACAAAAGGACCCAGGTTACATTGTTATTGACGTCAAC GCTGG | SEQ ID NO:2 |
| hox2 | CGAAATCGATGTTGGTACCAATCCATCTTCTGTTGAAACCTTGCTTCATGGATGGC AATCTTGGGTCAGGCTTGTCTGGAGTACCAGCGTTGACGTCAATAACAATG | SEQ ID NO:3 |
| hox3 | GATTGGTACCAACATCGATTTCGTTTACGTCGTTTACACTCCACAAGGTGCTTGTA CTGCTTGGACAGAGCTATGGAAAAGTGTTCTCCAGGTACCGTCAGAATC | SEQ ID NO:4 |
| hox4 | TTCAACCAAACCAGTAACGTTGATGATAGCCTTGACACATTCGTCGAAAACGAAGT CTTCGTAACAGTGACCACCAGAAACGATTCTGACGGTACCTGGAGAACAC | SEQ ID NO:5 |
| hox5 | ATCAACGTTACTGGTTTGGTTGAATCTGGTTACGACGACGATAGAGGTTACTTCGT CTCTTCCGGTGACACCAACTGGGGTTCCTTCAAGACCTTGTTCAGAGACCACGGTA GAGTTTTG | SEQ ID NO:6 |
| hox6 | CAAACCGTGCAATCTGGCCAAAATACCGTCACCTCCACCGACAATGTGACCACCCA AACCGACGGAGTAACAGGAACCACCTGGCAAAACTCTACCGTGGTCTCTGAAC | SEQ ID NO:7 |
| hox7 | TTTGGCCAGATTGCACGGTTTGCCAGTCGATTGGTTATCCGGTGTTGAAGTTGTCG TTAAGCCAGTCTTGACCGAAGACTCTGTTCTTAAGTACGTTCACAAGGATTCC | SEQ ID NO:8 |
| hox8 | GGCAAATCCTTGAAGTAGTATTTGGTGATAATACCGAAGTTACCTCCACCTCCACC AGTGTGAGCCCAAAACAACTCACCGTCGTTACCTTCGGAATCCTTGTGAACGTACT TAAG | SEQ ID NO:9 |
| hox9 | CAAATACTACTTCAAGGATTTGCCAATGTCTCCAAGAGGTGTCATCGCTTCTAACT TACACTTCTCTTGGGACGGTTTCACTAGAGATGCCTTGCAAGATTTGTTGACTAAG TACTTC | SEQ ID NO:10 |
| hox10 | GGAGGTATACAAGTACATGACGAACTCTTCAGCTGCTTGGTGGAAGATTTGGAACT TACCAACAGTATTCTTCCAATCACATCTAGCCAACTTGAAGTACTTAGTCAACAAA TCTTGC | SEQ ID NO:11 |
| hox11 | ATCTTCCATCAGGCAGCTGAAGAGTTTGTTATGTACTTGTATACATCCTACTCTAA CGACGCCGAGAGAGAAGTTGCCCAAGACAGACACTATCAT | SEQ ID NO:12 |
| hox12 | GAAAGGAGCCCAACCAGCTAGACCACCAAGAGCTTTGGTAGGCTCGCATGTTTTGT AGATCTGTTCAATGTCAGCCTCCAAATGATAGTGTCTGTCTTGGGC | SEQ ID NO:13 |
| hox13 | GCTGGTTGGGCTCCTTTCCCTGTTAGACCTAGACCTAGACACACATCCAAGACTTC TTATA TGCATGACGAGACTATGGACTACCCTTTC | SEQ ID NO:14 |

| | | |
|---|---|---|
| hox14 | AATCTGGAAGTCTGGAAAGTCCTTGATCATGTAAGCAGACTTGTACTTACCTCTCT<br>GATTAGGACCGGAACCGTTGATAGTCTCAGTCAAAGCGTAGAAAGGGTAGTCCATA<br>GTCTCGTC | SEQ ID NO:15 |
| hox15 | GACTTTCCAGACTTCCAGATTGATGTTATCTGGAAATACCTTACTGAGGTTCCTGA<br>CGGTTTGACTAGTGCCGAAATGAAGGATGCTCTTCTTCAGGTTGATATGTTC | SEQ ID NO:16 |
| hox16 | CTTGTCTTCTTCCTGCCAGTATGTCTGGTACTGCAGTTTGATGATGTACTCTCTCT<br>GAGCAACTGCAGTAGCATCCCAAACAACCTTGTGAATCTCACCACCGAACATATCA<br>ACCTGAAGAAGAGC | SEQ ID NO:17 |
| hox17 | ACATACTGGCAGGAAGAAGACAAGGATGCAGTTAACTTGAAGTGGATTAGAGACTT<br>TTACGAGGAGATGTATGAGCCTTATGGTGGTGTTCCAGACCCTAACACTCAG | SEQ ID NO:18 |
| hox18 | GGCACCATACTTACCGTTCTTCCAGTTGTTCAAGTCAACATCAGGGTAGTTGAAGT<br>AGCATCCCTCAAAAACACCTTTACCACTCTCAACCTGAGTGTTAGGGTCTGGAAC | SEQ ID NO:19 |
| hox19 | AAGAACGGTAAGTATGGTGCCTTGGAACTTTACTTTTTGGGTAACCTGAACAGATT<br>GATCAAGGCCAAATGGTTGTGGGATCCTAACGAGATCTTCACAAACAAACAGTCTA<br>TCCCT | SEQ ID NO:20 |
| hox20 | GAATTCCGCGGCCGCCTACTATTTAGTCTGCTTAGGCTCCTTAAGAGGTTTAGTAG<br>GGATAGACTGTTTGTTTGTGAA | SEQ ID NO:21 |
| *Bgl*II-*Nsi*I wt-KEX2, sense | GATCTACAAAACATGCGAGCCTACCAAAGCTCTTGGTGGTCATGCTGGTTGGGCTC<br>CTTTCCCTGTTAGACCTAGAAAGAGACACACATCCAAGACTTCTTATATGCA | SEQ ID NO:22 |
| *Bgl*II-*Nsi*I wt-KEX2, anti-sense | TATAAGAAGTCTTGGATGTGTGTCTCTTTCTAGGTCTAACAGGGAAAGGAGCCCAA<br>CCAGCATGACCACCAAGAGCTTTGGTAGGCTCGCATGTTTTGTA | SEQ ID NO:23 |
| MDTRS-sense | CGAAGCATGGACACTAGATC | SEQ ID NO:24 |
| MDTRS-antisense | CATGGATCTAGTGTCCATGCTT | SEQ ID NO:25 |
| MEAEA-sense | CGAAGCATGGAGGCTGAGGC | SEQ ID NO:26 |
| MEAEA-antisense | CATGGCCTCAGCCTCCATGCTT | SEQ ID NO:27 |
| -SKL sense | GATCCTAACGAGATCTTCACAAACAAACAGTCTATCCCTACTAAACCTCTTAAGGA<br>GCCTAAGCAGTCTAAGTTGTAATAGGC | SEQ ID NO:28 |
| -SKL antisense | GGCCGCCTATTACAACTTAGACTGCTTAGGCTCCTTAAGAGGTTTAGTAGGGATAG<br>ACTGTTTGTTTGTGAAGATCTCGTTAG | SEQ ID NO:29 |

The proximal half of the synthetic hexose oxidase gene was synthesized using the oligonucleotides hox1 through hox10 (Table 1), each with a length between 100 and 120 base pairs (bp) as primers in hot start polymerase chain reaction (PCR) using the proofreading, thermostable DNA polymerase *Pwo* (Boehringer). A concentration of 0.1 µM of each of the ten oligonucleotides was used in a 100 µl PCR reaction containing 2 mM $MgSO_4$. Hot start was performed by heating the mixture of oligonucleotides, buffer and $MgSO_4$ to 90°C before dNTP (250 µM) and *Pwo* polymerase (2.5 units) was added. Up to 40 cycles of PCR was conducted with a profile of 94°C for 30 seconds, 57°C for 1 minute and 72°C for 1 minute. At the end of the PCR a 10 minutes elongation step at 72°C was included. Analysis of the products from this PCR in agarose gel electrophoresis showed a smear of DNA bands ranging in size from about 100 bp to about 850 bp. This first PCR was reamplified in a second PCR. A volume of 2 µl from the first PCR was used as template in PCR with the oligonucleotides hox1 and hox10 (1 µM each) as primers. The reaction mixture contained 200 µM dNTP, 2.5 mM $MgCl_2$, and 2 units of AmpliTaq® (Perkin-Elmer Cetus). The PCR conditions were: 94°C for 2 minutes, then 30 cycles of PCR with the profile 94°C for 30 seconds, 60°C for 1 minute and 72°C for 45 seconds. A 10 minutes elongation step at 72°C was included at the end. Analysis of this second PCR in agarose gel electrophoresis showed the presence of a 850 bp DNA band. This 850 bp band was purified from the gel (QIAEX™, QIAGEN) and cloned into the vector pCR®2.1 (Invitrogen).

An analysis of the amino acid sequence of the C-terminal part of the hexose oxidase revealed a putative cleavage site for the KEX2 protease, -$Lys_{332}$-$Arg_{333}$-. Since yeast cells, including *P. pastoris*, contain a KEX2-like protease that has the potential for modification of secreted proteins, the DNA sequence for the C-terminal part of hexose oxidase was modified to give a change in the KEX2 recognition site to -$Pro_{332}$-$Arg_{333}$-.

The distal part of the hexose oxidase gene was synthesized as two fragments, each of a size of 530 bp. Two PCR reactions were performed using 6 primers at a time. The PCR reaction 1 contained the oligonucleotides hox11 through hox16 and PCR reaction 2 contained oligonuleotides hox15 through hox 20 (Table 1). The PCR amplification reactions were performed using 0.1 µM of each of the primers, 250 µM of each dNTP, 2 mM $MgSO_4$ and 2.5 units of *Pfu* DNA polymerase from *Pyrococcus furiosus* (Stratagen) at a reaction volume of 100 µl. The cycling parameters for the 2 PCR reactions using *Pfu* polymerase included a 1 minute denaturation step at 95°C folllowed by 30 cycles of PCR: 94°C for 1 minute, 55°C for 1 minute and 72°C for 1 minute. This was followed by an elongation step at 72°C.

Analysis of the PCR products from the two PCR reactions by agarose gel electrophoresis showed the presence of two DNA fragments each of a length of about 530 bp. The two fragments were purified and cloned into the vector pCR®-Blunt (Invitrogen). The cloned hexose oxidase gene fragments were sequenced using primers flanking the multiple cloning sites (M13 reverse primer and T7 promoter primer). The sequencing results verified that the constructed gene fragments contained the correct sequence.

The three parts of the synthetic hexose oxidase gene were combined as shown in Fig. 1. The proximal half part was purified on an agarose gel as a *Nco*I-*Pvu*II fragment, the middle part as a *Pvu*II-*Spe*I fragment and the distal part containing the changed KEX2 site as a *Spe*I-*Not*I fragment. The purified fragments were ligated and cloned into the vector pSCREEN (Novagen) resulting in plasmid pUPO314 (Fig.2).

A synthetic hexose oxidase gene with the wild type (wt) KEX2 recognition sequence, i.e. - $Lys_{332}$-$Arg_{333}$ - was constructed as illustrated in Fig. 3. Plasmid pUPO314 was restricted with the enzymes *Nco*I and *Bgl*II in one reaction and with *Nco*I and *Nsi*I in another. The resulting DNA fragments with the size of about 915 bp and about 4.7 kb, respectively, were purified and ligated with a 106 bp synthetic oligonucleotide, *Bgl*II-*Nsi*I wt-KEX2 (Table 1) encoding the sequence between the *Bgl*II and *Nsi*I sites including the wild type amino acid codon at - $Lys_{332}$-$Arg_{333}$-. This plasmid with the combined synthetic gene encoding the wild type hexose oxidase was designated pUPO316. The identity of the wild type synthetic hexose oxidase gene was confirmed by DNA-sequencing. Table 2 shows the nucleotide sequence of the synthetic hexose oxidase gene encoding the wild type enzyme.

Table 2. Nucleotide sequence of the synthetic hexose oxidase gene (SEQ ID NO:30) and the corresponding amino acid sequence of hexose oxidase (SEQ ID NO:31)

```
ATG GCT ACT TTG CCA CAA AAG GAC CCA GGT TAC ATT GTT ATT GAC GTC      48
Met Ala Thr Leu Pro Gln Lys Asp Pro Gly Tyr Ile Val Ile Asp Val
  1               5                  10                  15

AAC GCT GGT ACT CCA GAC AAG CCT GAC CCA AGA TTG CCA TCC ATG AAG      96
Asn Ala Gly Thr Pro Asp Lys Pro Asp Pro Arg Leu Pro Ser Met Lys
                 20                  25                  30

CAA GGT TTC AAC AGA AGA TGG ATT GGT ACC AAC ATC GAT TTC GTT TAC     144
Gln Gly Phe Asn Arg Arg Trp Ile Gly Thr Asn Ile Asp Phe Val Tyr
             35                  40                  45

GTC GTT TAC ACT CCA CAA GGT GCT TGT ACT GCT TTG GAC AGA GCT ATG     192
Val Val Tyr Thr Pro Gln Gly Ala Cys Thr Ala Leu Asp Arg Ala Met
     50                  55                  60

GAA AAG TGT TCT CCA GGT ACC GTC AGA ATC GTT TCT GGT GGT CAC TGT     240
Glu Lys Cys Ser Pro Gly Thr Val Arg Ile Val Ser Gly Gly His Cys
 65                  70                  75                  80

TAC GAA GAC TTC GTT TTC GAC GAA TGT GTC AAG GCT ATC ATC AAC GTT     288
Tyr Glu Asp Phe Val Phe Asp Glu Cys Val Lys Ala Ile Ile Asn Val
                 85                  90                  95

ACT GGT TTG GTT GAA TCT GGT TAC GAC GAC GAT AGA GGT TAC TTC GTC     336
Thr Gly Leu Val Glu Ser Gly Tyr Asp Asp Asp Arg Gly Tyr Phe Val
            100                 105                 110

TCT TCC GGT GAC ACC AAC TGG GGT TCC TTC AAG ACC TTG TTC AGA GAC     384
Ser Ser Gly Asp Thr Asn Trp Gly Ser Phe Lys Thr Leu Phe Arg Asp
        115                 120                 125

CAC GGT AGA GTT TTG CCA GGT GGT TCC TGT TAC TCC GTC GGT TTG GGT     432
His Gly Arg Val Leu Pro Gly Gly Ser Cys Tyr Ser Val Gly Leu Gly
    130                 135                 140
```

```
GGT CAC ATT GTC GGT GGA GGT GAC GGT ATT TTG GCC AGA TTG CAC GGT     480
Gly His Ile Val Gly Gly Gly Asp Gly Ile Leu Ala Arg Leu His Gly
145                 150                 155                 160

TTG CCA GTC GAT TGG TTA TCC GGT GTT GAA GTT GTC GTT AAG CCA GTC     528
Leu Pro Val Asp Trp Leu Ser Gly Val Glu Val Val Val Lys Pro Val
                165                 170                 175

TTG ACC GAA GAC TCT GTT CTT AAG TAC GTT CAC AAG GAT TCC GAA GGT     576
Leu Thr Glu Asp Ser Val Leu Lys Tyr Val His Lys Asp Ser Glu Gly
            180                 185                 190

AAC GAC GGT GAG TTG TTT TGG GCT CAC ACT GGT GGA GGT GGA GGT AAC     624
Asn Asp Gly Glu Leu Phe Trp Ala His Thr Gly Gly Gly Gly Gly Asn
        195                 200                 205

TTC GGT ATT ATC ACC AAA TAC TAC TTC AAG GAT TTG CCA ATG TCT CCA     672
Phe Gly Ile Ile Thr Lys Tyr Tyr Phe Lys Asp Leu Pro Met Ser Pro
    210                 215                 220

AGA GGT GTC ATC GCT TCT AAC TTA CAC TTC TCT TGG GAC GGT TTC ACT     720
Arg Gly Val Ile Ala Ser Asn Leu His Phe Ser Trp Asp Gly Phe Thr
225                 230                 235                 240

AGA GAT GCC TTG CAA GAT TTG TTG ACT AAG TAC TTC AAG TTG GCT AGA     768
Arg Asp Ala Leu Gln Asp Leu Leu Thr Lys Tyr Phe Lys Leu Ala Arg
                245                 250                 255

TGT GAT TGG AAG AAT ACT GTT GGT AAG TTC CAA ATC TTC CAC CAA GCA     816
Cys Asp Trp Lys Asn Thr Val Gly Lys Phe Gln Ile Phe His Gln Ala
            260                 265                 270

GCT GAA GAG TTT GTT ATG TAC TTG TAT ACA TCC TAC TCT AAC GAC GCC     864
Ala Glu Glu Phe Val Met Tyr Leu Tyr Thr Ser Tyr Ser Asn Asp Ala
        275                 280                 285

GAG AGA GAA GTT GCC CAA GAC AGA CAC TAT CAT TTG GAG GCT GAC ATT     912
Glu Arg Glu Val Ala Gln Asp Arg His Tyr His Leu Glu Ala Asp Ile
    290                 295                 300

GAA CAG ATC TAC AAA ACA TGC GAG CCT ACC AAA GCT CTT GGT GGT CAT     960
Glu Gln Ile Tyr Lys Thr Cys Glu Pro Thr Lys Ala Leu Gly Gly His
305                 310                 315                 320
```

```
GCT GGT TGG GCT CCT TTC CCT GTT AGA CCT AGA AAG AGA CAC ACA TCC     1008
Ala Gly Trp Ala Pro Phe Pro Val Arg Pro Arg Lys Arg His Thr Ser
                325                 330                 335

AAG ACT TCT TAT ATG CAT GAC GAG ACT ATG GAC TAC CCT TTC TAC GCT     1056
Lys Thr Ser Tyr Met His Asp Glu Thr Met Asp Tyr Pro Phe Tyr Ala
            340                 345                 350

TTG ACT GAG ACT ATC AAC GGT TCC GGT CCT AAT CAG AGA GGT AAG TAC     1104
Leu Thr Glu Thr Ile Asn Gly Ser Gly Pro Asn Gln Arg Gly Lys Tyr
        355                 360                 365

AAG TCT GCT TAC ATG ATC AAG GAC TTT CCA GAC TTC CAG ATT GAT GTT     1152
Lys Ser Ala Tyr Met Ile Lys Asp Phe Pro Asp Phe Gln Ile Asp Val
    370                 375                 380

ATC TGG AAA TAC CTT ACT GAG GTT CCT GAC GGT TTG ACT AGT GCC GAA     1200
Ile Trp Lys Tyr Leu Thr Glu Val Pro Asp Gly Leu Thr Ser Ala Glu
385                 390                 395                 400

ATG AAG GAT GCT CTT CTT CAG GTT GAT ATG TTC GGT GGT GAG ATT CAC     1248
Met Lys Asp Ala Leu Leu Gln Val Asp Met Phe Gly Gly Glu Ile His
                405                 410                 415

AAG GTT GTT TGG GAT GCT ACT GCA GTT GCT CAG AGA GAG TAC ATC ATC     1296
Lys Val Val Trp Asp Ala Thr Ala Val Ala Gln Arg Glu Tyr Ile Ile
            420                 425                 430

AAA CTG CAG TAC CAG ACA TAC TGG CAG GAA GAA GAC AAG GAT GCA GTT     1344
Lys Leu Gln Tyr Gln Thr Tyr Trp Gln Glu Glu Asp Lys Asp Ala Val
        435                 440                 445

AAC TTG AAG TGG ATT AGA GAC TTT TAC GAG GAG ATG TAT GAG CCT TAT     1392
Asn Leu Lys Trp Ile Arg Asp Phe Tyr Glu Glu Met Tyr Glu Pro Tyr
    450                 455                 460

GGT GGT GTT CCA GAC CCT AAC ACT CAG GTT GAG AGT GGT AAA GGT GTT     1440
Gly Gly Val Pro Asp Pro Asn Thr Gln Val Glu Ser Gly Lys Gly Val
465                 470                 475                 480
```

```
TTT GAG GGA TGC TAC TTC AAC TAC CCT GAT GTT GAC TTG AAC AAC TGG    1488
Phe Glu Gly Cys Tyr Phe Asn Tyr Pro Asp Val Asp Leu Asn Asn Trp
                485                 490                 495

AAG AAC GGT AAG TAT GGT GCC TTG GAA CTT TAC TTT TTG GGT AAC CTG    1536
Lys Asn Gly Lys Tyr Gly Ala Leu Glu Leu Tyr Phe Leu Gly Asn Leu
            500                 505                 510

AAC AGA TTG ATC AAG GCC AAA TGG TTG TGG GAT CCT AAC GAG ATC TTC    1584
Asn Arg Leu Ile Lys Ala Lys Trp Leu Trp Asp Pro Asn Glu Ile Phe
        515                 520                 525

ACA AAC AAA CAG TCT ATC CCT ACT AAA CCT CTT AAG GAG CCT AAG CAG    1632
Thr Asn Lys Gln Ser Ile Pro Thr Lys Pro Leu Lys Glu Pro Lys Gln
    530                 535                 540

ACT AAA TAG TAG
Thr Lys  stop
545
```

EXAMPLE 3

**Construction of *Pichia pastoris* expression vectors containing the synthetic hexose gene**

The synthetic hexose oxidase genes from pUPO314 and pUPO316 were further subcloned into the *P. pastoris* expression vector pPIC3 (Invitrogen) as shown in Fig. 4.

The synthetic hexose oxidase genes from pUPO314 and pUPO316 were inserted into the *Bam*HI and *Not*I sites of the plasmid pPIC3. As shown in Fig. 4, plasmids pUPO314 and pUPO316 were restricted with *Nco*I and the ends were filled out with DNA polymerase I, Klenow fragment. Following heat inactivation of the polymerase, pUPO314 and pUPO316 were restricted with the enzyme *Not*I and the synthetic hexose oxidase genes were purified on blunt end to *Not*I DNA fragments. The fragments were inserted into vector pPIC3 restricted with the enzyme *Bam*HI, treated with Mung Bean Nuclease and finally restricted with *Not*I. The resulting hexose oxidase expression plasmids were designated pUPO346 and pUPO349, respectively. The DNA sequence upstream of the translation initiation ATG codon (in bold) was: TTATTCGAAGC **ATG** (SEQ ID NO:32)

An alternative procedure for subcloning the synthetic hexose oxidase gene in pPIC3 was carried out by inserting the hexose oxidase gene from pUPO314 into the *Nco*I site of plasmid pPIC3. The synthetic hexose oxidase gene from pUPO314 was purified on a *Nco*I-*Not*I fragment and inserted into the pPIC3 restricted with *Not*I and partially restricted with *Nco*I. DNA sequence analysis of the resulting expression vector, pUPO340, showed the following DNA sequence upstream of the translation initiation ATG codon (in bold): GGATCCAAACC **ATG** (SEQ ID NO:33).

Samples of *P. pastoris* KM71 containing plasmids pUPO340, pUPO346 and pUPO349, respectively were deposited under the Budapest Treaty with the Deutsche Sammlung von Mikroorganismen und Zellkulturen (DSMZ), Maschroder Weg 1b, D-38124 Braunschweig, Germany on November 25, 1998 under the accession numbers DSM 12534, 12530 and 12531, respectively.

EXAMPLE 4

**Expression of recombinant hexose oxidase in *Pichia pastoris***

The hexose oxidase expression plasmids pUPO340, pUPO346 and pUPO349 were transformed into *P. pastoris* strain KM71 (genotype *his4, aox*::*ARG4*) as described by Stougaard and Hansen (1996). Cultures of *P. pastoris* containing pUPO340, pUPO346 or pUPO349 or the plasmid pUPO153 harbouring the hexose oxidase gene with the native *Chondrus crispus* codon usage (Stougaard and Hansen, 1996) were compared in shake flask cultures. Growth conditions and induction of hexose oxidase expression with methanol was as described by Stougaard and Hansen, (1996). Samples of the cultures were withdrawn and analysed for the presence of hexose oxidase. The cells were pelleted by centrifugation, resuspended in 50 mM Tris, pH 7.5, 10% glycerol containing approximately 1/3 volume of glassbeads (diameter 212 to 300 µm) and ruptured by sonication in Transsonic Digital, Elma or in Fast Prep FP120, BIO101, Savant Instruments. Cell membranes and other insoluble materials were pelleted and the supernatant was analysed by SDS-PAGE and Western blot analysis as described in Example 1.

Figs. 5 and 6 show Western blot analyses of proteins in intracellular extracts from *P. pastoris* KM71 containing pUPO153, pUPO340, pUPO346 and pUPO349, respectively.

In one set of experiments the expression level of pUPO153 (gene with native, algal codon usage) was compared to that of pUPO340 (synthetic gene) (Fig. 5). Western blot analysis of cell-extracts from induced cells harvested 1, 2, and 3 days after induction with methanol showed that the synthetic hexose oxidase gene from pUPO340 was expressed more efficiently than the native gene, irrespective of the time after induction.

In another set of experiments, plasmids pUPO153, pUPO340, pUPO346 and pUPO349 were linearized with the restriction enzyme *Sal*I before transformation into *P. pastoris* KM71. Transformants grown in shake flasks were analysed for the expression of hexose oxidase by Western blot analysis as described above. Fig. 6 shows that cell extracts isolated at day 2 after induction contained different amounts of recombinant hexose oxidase. In the Western blot experiment shown in Fig. 6, cell extracts from pUPO153 transformants contained very low amounts of hexose oxidase (although hexose oxidase enzyme activity could be measured), pUPO340 transformants contained some hexose oxidase, whereas pUPO346 and pUPO349 transformants were the most efficient hexose oxidase producers. Thus, it was concluded that the DNA-sequence upstream of the translation codon in pUPO346 and pUPO349 resulted in the highest expression efficiency.

EXAMPLE 5

**Fermentation of *Pichia pastoris* containing pUPO349**

Transformants of *P. pastoris* KM71 containing pUPO349 were analysed in fermentation experiments in a 10 liter fermentor (LH Fermentation). Media and growth conditions were as recomended in *Pichia* Fermentation Process Guidelines (Invitrogen): 10 liters of basal salt medium containing 4% (w/v) glycerol and 4.35 ml $PTM_1$ trace salts/liter were inoculated with 500 ml of an overnight culture having a cell density of $OD_{600}$ = 3. Cells were grown at 30°C and pH 5.0 (stirring at 1000 rpm, gas flow 50 l/min) until all the glycerol was consumed. The batch growth phase was followed by a four hours limited glycerol feed phase. Induction was initiated by feeding methanol containing 12 ml $PTM_1$ trace salts per liter methanol. Methanol feed was continued for 72 hours. Throughout this phase the concentration of methanol in the culture was determined using HPLC analysis and the feed rate was regulated to keep methanol concentration between 0.1-1.0%. After the methanol feed phase the fermentation was terminated and the cells were harvested by centrifugation. The cells were resuspended in an equal volume of 50 mM Tris pH 7.5 and the cell suspension was passed through a Z+ cell disrupter (Constart Systems) three times to obtain efficient lysis of the cells. The cell lysate was centrifuged and the supernatant (intracellular fraction) was collected. The intracellular fraction and the growth medium cleared of cells by centrifugation (extracellular fraction) were analysed by SDS-PAGE as described above (Fig. 7).

The level of hexose oxidase expressed in *P. pastoris* was determined by comparing the intensities of hexose oxidase bands with those of marker bands in SDS-PAGE gels. Fig. 7 shows that the intensity of the hexose oxidase band in the intracellular extract (lane 2) is comparable to the intensity of the marker protein bands applied at a concentration of 0.5 µg per band (lane 1). Since the 0.5 µg hexose oxidase observed in Fig. 7, lane 2, corresponded to 2 µl cell culture the intracellular concentration of hexose oxidase was calculated to be about 250 mg per liter.

Although plasmid pUPO349 does not contain signals for extracellular translocation, recombinant hexose oxidase was detected in extracellular extracts. A volume of 100 µl extracellular extract was concentrated by standard TCA precipitation and analysed on the same SDS-PAGE gel as intracellular extract and marker proteins (Fig. 7). By comparing the intensities of Coomassie Brilliant Blue stained bands in Fig. 7, lanes 1, 2, and 3, it can be calculated that the concentration of recombinant hexose oxidase in the medium was at least 5 mg per liter. This concentration corresponds well to a calculation based on hexose oxidase enzyme activity. The assay which was essentially as described by Sullivan and Ikawa (1973) and Hansen and Stougaard (1997) showed that the crude, extracellular extract contained about 104 units of hexose oxidase per ml. Assuming a specific activity of 16,000 units per mg purified protein (Hansen and Stougaard, 1997) it could be calculated that the concentration of recombinant hexose oxidase in the extracellular extract was at least 6.5 mg per liter.

EXAMPLE 6

**Construction and expression of an N-terminally extended synthetic hexose oxidase gene**

Experiments were carried out in order to investigate whether the hexose oxidase could be extended with an N-terminal tag without the enzyme loosing its activity. Therefore, expression vectors containing the synthetic hexose oxidase gene with five extra amino acid residues attached to the N-terminus were constructed through the consecutive steps shown in Figs. 8a and 8b. The first step involved the construction of a vector containing a wild type hexose oxidase sequence containing an *Nco*I site at the ATG translation initiation site. This was done by digesting plasmids pUPO340 and pUPO349 with the enzyme *Sca*I. The small *Sca*I fragment from the digestion of pUPO340 was ligated to the large *Sca*I fragment from the pUPO349 digestion resulting in plasmid pUPO427. In plasmid pUPO427 two restriction sites, *Bst*BI and *Nco*I, were located a few bases upstream of and at the translation site of the synthetic hexose oxidase gene, respectively. These sites were suited for the insertion of synthetic oligonucleotides causing an N-terminal extension of hexose oxidase.

However, plasmid pUPO427 contained two sites for each of the restriction enzymes *Bst*BI and *Nco*I. Therefore, the hexose oxidase gene was cloned on a *Sac*I-*Not*I fragment in the cloning vector pBluescript KS (Stratagene) similarly restricted with *Sac*I and *Not*I. This resulted in plasmid pUPO437 containing unique sites for the restriction enzymes *Bst*BI and *Nco*I.

Synthetic oligonucleotides with *Bst*BI and *Nco*I cohesive ends (Table 1) containing codons for the amino acid sequences Met-Asp-Thr-Arg-Ser- and Met-Glu-Ala-Glu-Ala- were inserted into pUPO437 restricted with the enzymes *Bst*BI and *Nco*I resulting in plasmids pUPO445 and pUPO446 (Fig. 8b). In pUPO445 the native hexose oxidase amino acid sequence is extended N-terminally with the amino acids Met-Asp-Thr-Arg-Ser and in pUPO446 with Met-Glu-Ala-Glu-Ala. Plasmids pUPO445 and pUPO446 were cut with the restriction enzymes *Sac*I and *Not*I and the fragments containing the extended hexose oxidase genes were purified and inserted into the *P. pastoris* expression vector pPIC3, resulting in pUPO453 and pUPO455, respectively.

Samples of *P. pastoris* KM71 containing plasmids pUPO453 and pUPO455, respectively were deposited unde the Budapest Treaty with the Deutsche Sammlung von Mikroorganismen und Zellkulturen (DSMZ), Maschroder Weg 1b, D-38124 Braunschweig, Germany on November 25, 1998 under the accession numbers DSM 12532 and 12533, respectively.

Plasmids pUPO453 and pUPO455 were linearized in a unique *Sal*I site and transformed into *P. pastoris* KM71. Transformants were analysed for production of N-terminally extended hexose oxidase. The presence of hexose oxidase was visualised using Western blot analysis and enzyme activity was shown in enzyme assays (fig. 9). The Western blot analysis shows that hexose oxidase N-terminally extended with the amino acids $Met_{NH2}$-Asp-Thr-Arg-Ser or with $Met_{NH2}$-Glu-Ala-Glu-Ala were expressed as efficient as the wild type gene. Enzyme activity measurements (Fig. 9B, lanes 2 and 3) showed that the activity of the extended hexose oxidase enzymes was similar to the activity of the wild type enzyme (Fig. 9B, lane 1).

EXAMPLE 7

**Construction and expression of a C-terminally extended synthetic hexose oxidase gene**

Similar to the experiments with N-terminal extensions, it was examined whether C-terminally extended hexose oxidase could be synthesized and if the tagged enzyme was active. Fig. 10 shows the construction of a *P. pastoris* vector containing a hexose oxidase gene C-terminally extended with a DNA fragment encoding the extra amino acids -Ser-Lys-$Leu_{COOH}$. Plasmid pUPO349 was restricted with the enzymes *Bam*HI and *Not*I, the large fragment was purified and ligated to a synthetic oligonucleotide (Table 1) encoding the hexose oxidase gene distal to the *Bam*HI site plus an additional DNA sequence encoding codons for the amino acids Ser-Lys-$Leu_{COOH}$. The resulting plasmid, pUPO387 was linearised and transformed into *P. pastoris* KM71.

A sample of *P. pastoris* KM71 containing plasmids pUPO387 was deposited under the Budapest Treaty with the Deutsche Sammlung von Mikroorganismen und Zellkulturen (DSMZ), Maschroder Weg 1b, D-38124 Braunschweig, Germany on November 25, 1998 under the accession number DSM 12535.

Transformants of *P. pastoris* KM71 containing pUPO387 were analysed for production of C-terminally extended hexose oxidase. Cells were grown as described and induced with methanol. Cells were harvested and opened and the hexose oxidase containing extract was analysed using Western blot analysis and enzyme activity assays. Fig. 9, lane 4 shows that cells containing pUPO387 expressed C-terminally extended hexose oxidase at least as efficient as the wild type construct, pUPO349 (Fig. 9A, lane 1). Enzyme assays showed that the recombinant hexose oxidase tagged with -Ser-Lys-$Leu_{COOH}$ was at least as active as the wild type enzyme expressed from the plasmid pUPO349 (Fig. 9B, lanes 4 and 1, respectively).

REFERENCES

Cregg, J.M., Vedvick, T.S. and Raschke, W.C. 1993. Recent advances in the expression of foreign genes in *Pichia pastoris*. BIO/TECHNOLOGY, **11**:905-910.

Hansen, O.C. and Stougaard, P. 1997. Hexose oxidase from the red alga *Chondrus crispus*: Purification, Molecular cloning, and expression in *Pichia pastoris*. Journal of Biological Chemistry, **272**:11581-11587.

Hoekema, A., Kastelein, R.A., Vasser, M. and de Boer, H.A. 1987. Codon replacement in the *PGK1* gene of *Saccharomyces cerevisiae*: Experimental approach to study the role of biased codon usage in gene expression. Molecular and Cellular Biology, **7**:2914-2924.

Hunkapiller, M.W., Lujan, U., Ostrander, F., and Hood, L.E. 1983. Isolation of proteins from polyacrylamide gels for amino acid sequence analysis. Methods in Enzymology, **91**:227-236.

Makrides, S.C. 1996. Strategies for achieving high-level expression of genes in *Escherichia coli*. Microbiological Reviews, **60**:512-538.

Provart, N. 1997. Prokaryotic protein,
http://invitrogen.com/bin/wwwboard/pichia/messages/454.html

Shu-Hsing Wu, Lagarias, J.C. 1996. The methylotrophic yeast *Pichia pastoris* synthesizes a functionally active chromophore precursor of the plant photoreceptor phytochrome. PNAS, **93**:8989-8994.

Sreekrishna, K. 1996. Codon usage in *Pichia*,
http://www.invitrogen.com/bin/wwwboard/pichia/messages/69.html

Stougaard, P. and Hansen, O.C. 1996. Recombinant hexose oxidase, A method of producing same and use of such enzyme. WO 96/40935.

Sullivan, J.D. and Ikawa, M. 1973. Purification and characterization of hexose oxidase from the red alga *Chondrus crispus*. Biochemica et Biophysica Acta, **309**:11-22.

Anonymous, *Pichia* Fermentation Process Guidelines, Invitrogen.

SEQUENCE LISTING

<110> Bioteknologisk Institut

<120> MODIFIED DNA SEQUENCE CODING FOR HEXOSE OXIDASE AND USE HEREOF

<130> 20976DK1

<160> 33

<170> FastSEQ for Windows Version 3.0

<210> 1
<211> 10
<212> PRT
<213> Artificial Sequence

<220>
<223> Hexose oxidase produced in Escherichia coli from the expression plasmid pUPO181 as described in the co-pending application WO 96/40935.

<400> 1
```
Ala Thr Leu Pro Gln Lys Asp Pro Gly Tyr
 1               5                  10
```

<210> 2
<211> 61
<212> DNA
<213> Artificial Sequence

<220>
<223> Synthetic oligonucleotide designed to synthesize a hexose oxidase gene with a codon usage possibly optimized for expression in P. pastoris.

<400> 2
```
actccatggc tactttgcca caaaaggacc caggttacat tgttattgac gtcaacgctg      60
g                                                                      61
```

<210> 3
<211> 107
<212> DNA
<213> Artificial Sequence

<220>
<223> Synthetic oligonucleotide designed to synthesize a hexose oxidase gene with a codon usage possibly optimized for expression in P. pastoris.

<400> 3
```
cgaaatcgat gttggtacca atccatcttc tgttgaaacc ttgcttcatg gatggcaatc      60
ttgggtcagg cttgtctgga gtaccagcgt tgacgtcaat aacaatg                  107
```

<210> 4
<211> 105
<212> DNA
<213> Artificial Sequence

<220>
<223> Synthetic oligonucleotide designed to synthesize a

hexose oxidase gene with a codon usage possibly optimized for expression in P. pastoris.

<400> 4
```
gattggtacc aacatcgatt tcgtttacgt cgtttacact ccacaaggtg cttgtactgc    60
ttggacagag ctatggaaaa gtgttctcca ggtaccgtca gaatc                   105
```

<210> 5
<211> 106
<212> DNA
<213> Artificial Sequence

<220>
<223> Synthetic oligonucleotide designed to synthesize a hexose oxidase gene with a codon usage possibly optimized for expression in P. pastoris.

<400> 5
```
ttcaaccaaa ccagtaacgt tgatgatagc cttgacacat tcgtcgaaaa cgaagtcttc    60
gtaacagtga ccaccagaaa cgattctgac ggtacctgga gaacac                  106
```

<210> 6
<211> 120
<212> DNA
<213> Artificial Sequence

<220>
<223> Synthetic oligonucleotide designed to synthesize a hexose oxidase gene with a codon usage possibly optimized for expression in P. pastoris.

<400> 6
```
atcaacgtta ctggtttggt tgaatctggt tacgacgacg atagaggtta cttcgtctct    60
tccggtgaca ccaactgggg ttccttcaag accttgttca gagaccacgg tagagttttg   120
```

<210> 7
<211> 109
<212> DNA
<213> Artificial Sequence

<220>
<223> Synthetic oligonucleotide designed to synthesize a hexose oxidase gene with a codon usage possibly optimized for expression in P. pastoris.

<400> 7
```
caaaccgtgc aatctggcca aaataccgtc acctccaccg acaatgtgac cacccaaacc    60
gacggagtaa caggaaccac ctggcaaaac tctaccgtgg tctctgaac               109
```

<210> 8
<211> 109
<212> DNA
<213> Artificial Sequence

<220>
<223> Synthetic oligonucleotide designed to synthesize a hexose oxidase gene with a codon usage possibly optimized for expression in P. pastoris.

<400> 8
```
tttggccaga ttgcacggtt tgccagtcga ttggttatcc ggtgttgaag ttgtcgttaa    60
gccagtcttg accgaagact ctgttcttaa gtacgttcac aaggattcc               109
```

<210> 9

<211> 116
<212> DNA
<213> Artificial Sequence

<220>
<223> Synthetic oligonucleotide designed to synthesize a hexose oxidase gene with a codon usage possibly optimized for expression in P. pastoris.

<400> 9
ggcaaatcct tgaagtagta tttggtgata ataccgaagt tacctccacc tccaccagtg 60
tgagcccaaa acaactcacc gtcgttacct tcggaatcct tgtgaacgta cttaag 116

<210> 10
<211> 118
<212> DNA
<213> Artificial Sequence

<220>
<223> Synthetic oligonucleotide designed to synthesize a hexose oxidase gene with a codon usage possibly optimized for expression in P. pastoris.

<400> 10
caaatactac ttcaaggatt tgccaatgtc tccaagaggt gtcatcgctt ctaacttaca 60
cttctcttgg gacggtttca ctagagatgc cttgcaagat ttgttgacta agtacttc 118

<210> 11
<211> 118
<212> DNA
<213> Artificial Sequence

<220>
<223> Synthetic oligonucleotide designed to synthesize a hexose oxidase gene with a codon usage possibly optimized for expression in P. pastoris.

<400> 11
ggaggtatac aagtacatga cgaactcttc agctgcttgg tggaagattt ggaacttacc 60
aacagtattc ttccaatcac atctagccaa cttgaagtac ttagtcaaca aatcttgc 118

<210> 12
<211> 96
<212> DNA
<213> Artificial Sequence

<220>
<223> Synthetic oligonucleotide designed to synthesize a hexose oxidase gene with a codon usage possibly optimized for expression in P. pastoris.

<400> 12
atcttccatc aggcagctga agagtttgtt atgtacttgt atacatccta ctctaacgac 60
gccgagagag aagttgccca agacagacac tatcat 96

<210> 13
<211> 102
<212> DNA
<213> Artificial Sequence

<220>
<223> Synthetic oligonucleotide designed to synthesize a hexose oxidase gene with a codon usage possibly optimized for expression in P. pastoris.

<400> 13
gaaaggagcc caaccagcta gaccaccaag agctttggta ggctcgcatg ttttgtagat 60
ctgttcaatg tcagcctcca aatgatagtg tctgtcttgg gc 102

<210> 14
<211> 90
<212> DNA
<213> Artificial Sequence

<220>
<223> Synthetic oligonucleotide designed to synthesize a hexose oxidase gene with a codon usage possibly optimized for expression in P. pastoris.

<400> 14
gctggttggg ctcctttccc tgttagacct agacctagac acacatccaa gacttcttat 60
atgcatgacg agactatgga ctaccctttc 90

<210> 15
<211> 120
<212> DNA
<213> Artificial Sequence

<220>
<223> Synthetic oligonucleotide designed to synthesize a hexose oxidase gene with a codon usage possibly optimized for expression in P. pastoris.

<400> 15
aatctggaag tctggaaagt ccttgatcat gtaagcagac ttgtacttac ctctctgatt 60
aggaccggaa ccgttgatag tctcagtcaa agcgtagaaa gggtagtcca tagtctcgtc 120

<210> 16
<211> 108
<212> DNA
<213> Artificial Sequence

<220>
<223> Synthetic oligonucleotide designed to synthesize a hexose oxidase gene with a codon usage possibly optimized for expression in P. pastoris.

<400> 16
gactttccag acttccagat tgatgttatc tggaaatacc ttactgaggt tcctgacggt 60
ttgactagtg ccgaaatgaa ggatgctctt cttcaggttg atatgttc 108

<210> 17
<211> 126
<212> DNA
<213> Artificial Sequence

<220>
<223> Synthetic oligonucleotide designed to synthesize a hexose oxidase gene with a codon usage possibly optimized for expression in P. pastoris.

<400> 17
cttgtcttct tcctgccagt atgtctggta ctgcagtttg atgatgtact ctctctgagc 60
aactgcagta gcatcccaaa caaccttgtg aatctcacca ccgaacatat caacctgaag 120
aagagc 126

<210> 18
<211> 108

<212> DNA
<213> Artificial Sequence

<220>
<223> Synthetic oligonucleotide designed to synthesize a hexose oxidase gene with a codon usage possibly optimized for expression in P. pastoris.

<400> 18
acatactggc aggaagaaga caaggatgca gttaacttga agtggattag agacttttac 60
gaggagatgt atgagcctta tggtggtgtt ccagacccta acactcag 108

<210> 19
<211> 111
<212> DNA
<213> Artificial Sequence

<220>
<223> Synthetic oligonucleotide designed to synthesize a hexose oxidase gene with a codon usage possibly optimized for expression in P. pastoris.

<400> 19
ggcaccatac ttaccgttct tccagttgtt caagtcaaca tcagggtagt tgaagtagca 60
tccctcaaaa acacctttac cactctcaac ctgagtgtta gggtctggaa c 111

<210> 20
<211> 117
<212> DNA
<213> Artificial Sequence

<220>
<223> Synthetic oligonucleotide designed to synthesize a hexose oxidase gene with a codon usage possibly optimized for expression in P. pastoris.

<400> 20
aagaacggta agtatggtgc cttggaactt tactttttgg gtaacctgaa cagattgatc 60
aaggccaaat ggttgtggga tcctaacgag atcttcacaa acaaacagtc tatccct 117

<210> 21
<211> 78
<212> DNA
<213> Artificial Sequence

<220>
<223> Synthetic oligonucleotide designed to synthesize a hexose oxidase gene with a codon usage possibly optimized for expression in P. pastoris.

<400> 21
gaattccgcg gccgcctact atttagtctg cttaggctcc ttaagaggtt tagtagggat 60
agactgtttg tttgtgaa 78

<210> 22
<211> 108
<212> DNA
<213> Artificial Sequence

<220>
<223> Synthetic oligonucleotide designed to synthesize a hexose oxidase gene with a codon usage possibly optimized for expression in P. pastoris.

<400> 22
gatctacaaa acatgcgagc ctaccaaagc tcttggtggt catgctggtt gggctccttt 60
ccctgttaga cctagaaaga gacacacatc caagacttct tatatgca 108

<210> 23
<211> 100
<212> DNA
<213> Artificial Sequence

<220>
<223> Synthetic oligonucleotide designed to synthesize a hexose oxidase gene with a codon usage possibly optimized for expression in P. pastoris.

<400> 23
tataagaagt cttggatgtg tgtctctttc taggtctaac agggaaagga gcccaaccag 60
catgaccacc aagagctttg gtaggctcgc atgttttgta 100

<210> 24
<211> 20
<212> DNA
<213> Artificial Sequence

<220>
<223> Synthetic oligonucleotide designed to synthesize a hexose oxidase gene with a codon usage possibly optimized for expression in P. pastoris.

<400> 24
cgaagcatgg acactagatc 20

<210> 25
<211> 22
<212> DNA
<213> Artificial Sequence

<220>
<223> Synthetic oligonucleotide designed to synthesize a hexose oxidase gene with a codon usage possibly optimized for expression in P. pastoris.

<400> 25
catggatcta gtgtccatgc tt 22

<210> 26
<211> 20
<212> DNA
<213> Artificial Sequence

<220>
<223> Synthetic oligonucleotide designed to synthesize a hexose oxidase gene with a codon usage possibly optimized for expression in P. pastoris.

<400> 26
cgaagcatgg aggctgaggc 20

<210> 27
<211> 22
<212> DNA
<213> Artificial Sequence

<220>
<223> Synthetic oligonucleotide designed to synthesize a

hexose oxidase gene with a codon usage possibly optimized for expression in P. pastoris.

<400> 27
```
catggcctca gcctccatgc tt                                                22
```

<210> 28
<211> 83
<212> DNA
<213> Artificial Sequence

<220>
<223> Synthetic oligonucleotide designed to synthesize a hexose oxidase gene with a codon usage possibly optimized for expression in P. pastoris.

<400> 28
```
gatcctaacg agatcttcac aaacaaacag tctatcccta ctaaacctct taaggagcct      60
aagcagtcta agttgtaata ggc                                               83
```

<210> 29
<211> 83
<212> DNA
<213> Artificial Sequence

<220>
<223> Synthetic oligonucleotide designed to synthesize a hexose oxidase gene with a codon usage possibly optimized for expression in P. pastoris.

<400> 29
```
ggccgcctat tacaacttag actgcttagg ctccttaaga ggtttagtag ggatagactg      60
tttgtttgtg aagatctcgt tag                                               83
```

<210> 30
<211> 1644
<212> DNA
<213> Artificial Sequence

<220>
<221> CDS
<222> (0)...(1644)

<223> Nucleotide sequence of synthetic hexose oxidase gene encoding wild type hexose oxidase

<400> 30
```
atg gct act ttg cca caa aag gac cca ggt tac att gtt att gac gtc       48
Met Ala Thr Leu Pro Gln Lys Asp Pro Gly Tyr Ile Val Ile Asp Val
 1               5                  10                  15

aac gct ggt act cca gac aag cct gac cca aga ttg cca tcc atg aag       96
Asn Ala Gly Thr Pro Asp Lys Pro Asp Pro Arg Leu Pro Ser Met Lys
            20                  25                  30

caa ggt ttc aac aga aga tgg att ggt acc aac atc gat ttc gtt tac      144
Gln Gly Phe Asn Arg Arg Trp Ile Gly Thr Asn Ile Asp Phe Val Tyr
        35                  40                  45

gtc gtt tac act cca caa ggt gct tgt act gct ttg gac aga gct atg      192
Val Val Tyr Thr Pro Gln Gly Ala Cys Thr Ala Leu Asp Arg Ala Met
    50                  55                  60

gaa aag tgt tct cca ggt acc gtc aga atc gtt tct ggt ggt cac tgt      240
```

```
Glu Lys Cys Ser Pro Gly Thr Val Arg Ile Val Ser Gly Gly His Cys
 65                 70                  75                  80

tac gaa gac ttc gtt ttc gac gaa tgt gtc aag gct atc atc aac gtt      288
Tyr Glu Asp Phe Val Phe Asp Glu Cys Val Lys Ala Ile Ile Asn Val
                85                  90                  95

act ggt ttg gtt gaa tct ggt tac gac gac gat aga ggt tac ttc gtc      336
Thr Gly Leu Val Glu Ser Gly Tyr Asp Asp Asp Arg Gly Tyr Phe Val
            100                 105                 110

tct tcc ggt gac acc aac tgg ggt tcc ttc aag acc ttg ttc aga gac      384
Ser Ser Gly Asp Thr Asn Trp Gly Ser Phe Lys Thr Leu Phe Arg Asp
        115                 120                 125

cac ggt aga gtt ttg cca ggt ggt tcc tgt tac tcc gtc ggt ttg ggt      432
His Gly Arg Val Leu Pro Gly Gly Ser Cys Tyr Ser Val Gly Leu Gly
    130                 135                 140

ggt cac att gtc ggt gga ggt gac ggt att ttg gcc aga ttg cac ggt      480
Gly His Ile Val Gly Gly Gly Asp Gly Ile Leu Ala Arg Leu His Gly
145                 150                 155                 160

ttg cca gtc gat tgg tta tcc ggt gtt gaa gtt gtc gtt aag cca gtc      528
Leu Pro Val Asp Trp Leu Ser Gly Val Glu Val Val Val Lys Pro Val
                165                 170                 175

ttg acc gaa gac tct gtt ctt aag tac gtt cac aag gat tcc gaa ggt      576
Leu Thr Glu Asp Ser Val Leu Lys Tyr Val His Lys Asp Ser Glu Gly
            180                 185                 190

aac gac ggt gag ttg ttt tgg gct cac act ggt gga ggt gga ggt aac      624
Asn Asp Gly Glu Leu Phe Trp Ala His Thr Gly Gly Gly Gly Gly Asn
        195                 200                 205

ttc ggt att atc acc aaa tac tac ttc aag gat ttg cca atg tct cca      672
Phe Gly Ile Ile Thr Lys Tyr Tyr Phe Lys Asp Leu Pro Met Ser Pro
    210                 215                 220

aga ggt gtc atc gct tct aac tta cac ttc tct tgg gac ggt ttc act      720
Arg Gly Val Ile Ala Ser Asn Leu His Phe Ser Trp Asp Gly Phe Thr
225                 230                 235                 240

aga gat gcc ttg caa gat ttg ttg act aag tac ttc aag ttg gct aga      768
Arg Asp Ala Leu Gln Asp Leu Leu Thr Lys Tyr Phe Lys Leu Ala Arg
                245                 250                 255

tgt gat tgg aag aat act gtt ggt aag ttc caa atc ttc cac caa gca      816
Cys Asp Trp Lys Asn Thr Val Gly Lys Phe Gln Ile Phe His Gln Ala
            260                 265                 270

gct gaa gag ttt gtt atg tac ttg tat aca tcc tac tct aac gac gcc      864
Ala Glu Glu Phe Val Met Tyr Leu Tyr Thr Ser Tyr Ser Asn Asp Ala
        275                 280                 285

gag aga gaa gtt gcc caa gac aga cac tat cat ttg gag gct gac att      912
Glu Arg Glu Val Ala Gln Asp Arg His Tyr His Leu Glu Ala Asp Ile
    290                 295                 300

gaa cag atc tac aaa aca tgc gag cct acc aaa gct ctt ggt ggt cat      960
Glu Gln Ile Tyr Lys Thr Cys Glu Pro Thr Lys Ala Leu Gly Gly His
305                 310                 315                 320

gct ggt tgg gct cct ttc cct gtt aga cct aga aag aga cac aca tcc     1008
```

```
Ala Gly Trp Ala Pro Phe Pro Val Arg Pro Arg Lys Arg His Thr Ser
                325                 330                 335

aag act tct tat atg cat gac gag act atg gac tac cct ttc tac gct      1056
Lys Thr Ser Tyr Met His Asp Glu Thr Met Asp Tyr Pro Phe Tyr Ala
            340                 345                 350

ttg act gag act atc aac ggt tcc ggt cct aat cag aga ggt aag tac      1104
Leu Thr Glu Thr Ile Asn Gly Ser Gly Pro Asn Gln Arg Gly Lys Tyr
        355                 360                 365

aag tct gct tac atg atc aag gac ttt cca gac ttc cag att gat gtt      1152
Lys Ser Ala Tyr Met Ile Lys Asp Phe Pro Asp Phe Gln Ile Asp Val
    370                 375                 380

atc tgg aaa tac ctt act gag gtt cct gac ggt ttg act agt gcc gaa      1200
Ile Trp Lys Tyr Leu Thr Glu Val Pro Asp Gly Leu Thr Ser Ala Glu
385                 390                 395                 400

atg aag gat gct ctt ctt cag gtt gat atg ttc ggt ggt gag att cac      1248
Met Lys Asp Ala Leu Leu Gln Val Asp Met Phe Gly Gly Glu Ile His
                405                 410                 415

aag gtt gtt tgg gat gct act gca gtt gct cag aga gag tac atc atc      1296
Lys Val Val Trp Asp Ala Thr Ala Val Ala Gln Arg Glu Tyr Ile Ile
            420                 425                 430

aaa ctg cag tac cag aca tac tgg cag gaa gaa gac aag gat gca gtt      1344
Lys Leu Gln Tyr Gln Thr Tyr Trp Gln Glu Glu Asp Lys Asp Ala Val
        435                 440                 445

aac ttg aag tgg att aga gac ttt tac gag gag atg tat gag cct tat      1392
Asn Leu Lys Trp Ile Arg Asp Phe Tyr Glu Glu Met Tyr Glu Pro Tyr
    450                 455                 460

ggt ggt gtt cca gac cct aac act cag gtt gag agt ggt aaa ggt gtt      1440
Gly Gly Val Pro Asp Pro Asn Thr Gln Val Glu Ser Gly Lys Gly Val
465                 470                 475                 480

ttt gag gga tgc tac ttc aac tac cct gat gtt gac ttg aac aac tgg      1488
Phe Glu Gly Cys Tyr Phe Asn Tyr Pro Asp Val Asp Leu Asn Asn Trp
                485                 490                 495

aag aac ggt aag tat ggt gcc ttg gaa ctt tac ttt ttg ggt aac ctg      1536
Lys Asn Gly Lys Tyr Gly Ala Leu Glu Leu Tyr Phe Leu Gly Asn Leu
            500                 505                 510

aac aga ttg atc aag gcc aaa tgg ttg tgg gat cct aac gag atc ttc      1584
Asn Arg Leu Ile Lys Ala Lys Trp Leu Trp Asp Pro Asn Glu Ile Phe
        515                 520                 525

aca aac aaa cag tct atc cct act aaa cct ctt aag gag cct aag cag      1632
Thr Asn Lys Gln Ser Ile Pro Thr Lys Pro Leu Lys Glu Pro Lys Gln
    530                 535                 540

act aaa tag tag                                                      1644
Thr Lys  *   *
545
```

<210> 31
<211> 546
<212> PRT
<213> Artificial Sequence

<220>
<223> Amino acid sequence of wild type hexose oxidase

<400> 31

```
Met Ala Thr Leu Pro Gln Lys Asp Pro Gly Tyr Ile Val Ile Asp Val
 1               5                  10                  15
Asn Ala Gly Thr Pro Asp Lys Pro Asp Pro Arg Leu Pro Ser Met Lys
            20                  25                  30
Gln Gly Phe Asn Arg Arg Trp Ile Gly Thr Asn Ile Asp Phe Val Tyr
        35                  40                  45
Val Val Tyr Thr Pro Gln Gly Ala Cys Thr Ala Leu Asp Arg Ala Met
    50                  55                  60
Glu Lys Cys Ser Pro Gly Thr Val Arg Ile Val Ser Gly Gly His Cys
65                  70                  75                  80
Tyr Glu Asp Phe Val Phe Asp Glu Cys Val Lys Ala Ile Ile Asn Val
                85                  90                  95
Thr Gly Leu Val Glu Ser Gly Tyr Asp Asp Asp Arg Gly Tyr Phe Val
            100                 105                 110
Ser Ser Gly Asp Thr Asn Trp Gly Ser Phe Lys Thr Leu Phe Arg Asp
        115                 120                 125
His Gly Arg Val Leu Pro Gly Gly Ser Cys Tyr Ser Val Gly Leu Gly
    130                 135                 140
Gly His Ile Val Gly Gly Gly Asp Gly Ile Leu Ala Arg Leu His Gly
145                 150                 155                 160
Leu Pro Val Asp Trp Leu Ser Gly Val Glu Val Val Val Lys Pro Val
                165                 170                 175
Leu Thr Glu Asp Ser Val Leu Lys Tyr Val His Lys Asp Ser Glu Gly
            180                 185                 190
Asn Asp Gly Glu Leu Phe Trp Ala His Thr Gly Gly Gly Gly Gly Asn
        195                 200                 205
Phe Gly Ile Ile Thr Lys Tyr Tyr Phe Lys Asp Leu Pro Met Ser Pro
    210                 215                 220
Arg Gly Val Ile Ala Ser Asn Leu His Phe Ser Trp Asp Gly Phe Thr
225                 230                 235                 240
Arg Asp Ala Leu Gln Asp Leu Leu Thr Lys Tyr Phe Lys Leu Ala Arg
                245                 250                 255
Cys Asp Trp Lys Asn Thr Val Gly Lys Phe Gln Ile Phe His Gln Ala
            260                 265                 270
Ala Glu Glu Phe Val Met Tyr Leu Tyr Thr Ser Tyr Ser Asn Asp Ala
        275                 280                 285
Glu Arg Glu Val Ala Gln Asp Arg His Tyr His Leu Glu Ala Asp Ile
    290                 295                 300
Glu Gln Ile Tyr Lys Thr Cys Glu Pro Thr Lys Ala Leu Gly Gly His
305                 310                 315                 320
Ala Gly Trp Ala Pro Phe Pro Val Arg Pro Arg Lys Arg His Thr Ser
                325                 330                 335
Lys Thr Ser Tyr Met His Asp Glu Thr Met Asp Tyr Pro Phe Tyr Ala
            340                 345                 350
Leu Thr Glu Thr Ile Asn Gly Ser Gly Pro Asn Gln Arg Gly Lys Tyr
        355                 360                 365
Lys Ser Ala Tyr Met Ile Lys Asp Phe Pro Asp Phe Gln Ile Asp Val
    370                 375                 380
Ile Trp Lys Tyr Leu Thr Glu Val Pro Asp Gly Leu Thr Ser Ala Glu
385                 390                 395                 400
Met Lys Asp Ala Leu Leu Gln Val Asp Met Phe Gly Gly Glu Ile His
                405                 410                 415
Lys Val Val Trp Asp Ala Thr Ala Val Ala Gln Arg Glu Tyr Ile Ile
            420                 425                 430
Lys Leu Gln Tyr Gln Thr Tyr Trp Gln Glu Glu Asp Lys Asp Ala Val
        435                 440                 445
Asn Leu Lys Trp Ile Arg Asp Phe Tyr Glu Glu Met Tyr Glu Pro Tyr
    450                 455                 460
Gly Gly Val Pro Asp Pro Asn Thr Gln Val Glu Ser Gly Lys Gly Val
```

```
465                 470                 475                 480
Phe Glu Gly Cys Tyr Phe Asn Tyr Pro Asp Val Asp Leu Asn Asn Trp
                485                 490                 495
Lys Asn Gly Lys Tyr Gly Ala Leu Glu Leu Tyr Phe Leu Gly Asn Leu
            500                 505                 510
Asn Arg Leu Ile Lys Ala Lys Trp Leu Trp Asp Pro Asn Glu Ile Phe
        515                 520                 525
Thr Asn Lys Gln Ser Ile Pro Thr Lys Pro Leu Lys Glu Pro Lys Gln
    530                 535                 540
Thr Lys
545
```

```
      <210> 32
      <211> 14
      <212> DNA
      <213> Artificial Sequence

      <220>
      <223> DNA sequence upstream of the translation
            initiation ATG codon

      <400> 32
ttattcgaag catg                                                       14

      <210> 33
      <211> 14
      <212> DNA
      <213> Artificial Sequence

      <220>
      <223> DNA sequence upstream of the translation
            initiation ATG codon

      <400> 33
ggatccaaac catg                                                       14
1


1
```

**Claims**

1. A nucleic acid fragment comprising a modified nucleotide sequence coding for a hexose oxidase, the fragment is obtainable by

   selecting a source organism naturally producing a hexose oxidase, isolating from said organism the nucleotide sequence coding for said naturally produced hexose oxidase and modifying said sequence, the thus modified nucleotide sequence is expressible in a host organism at a level which, relative to the level at which the non-modified nucleotide sequence is expressed in said host organism under substantially identical conditions, is at least 10% higher, including a nucleic acid fragment which is obtained by

   (i) selecting a source organism naturally producing a hexose oxidase, isolating from said organism the nucleotide sequence coding for said naturally produced hexose oxidase and determing its nucleotide sequence,

   (ii) selecting a host organism,

(iii) determining for said host organism the frequency of nucleotide triplets (codons) coding for individual amino acids (codon usage), and

(iv) modifying the isolated nucleotide sequence by substituting at least one codon coding for an amino acid with a different codon coding for the same amino acid including such different codon that is used naturally by said host organism at a higher frequency than the codon being substituted, the nucleotide sequence coding for said naturally produced hexose oxidase preferably being isolated from an algal species selected from the group consisting of *Chondrus crispus*, *Iridophycus flaccidum* and *Euthora cristata* such as a nucleic acid fragment where the nucleotide sequence coding for said naturally produced hexose oxidase is SEQ ID NO:30 as shown in WO 96/40935 or a part thereof.

**2.** A nucleic acid fragment according to claim 1 where the sequence coding for hexose oxidase is modified to comprise at least one codon that codes for an amino acid residue which does not occur in the naturally produced hexose oxidase.

**3.** A nucleic acid fragment according to claim 1 that is derived from a nucleic acid fragment comprising at least part of the nucleotide sequence coding for the hexose oxidase of SEQ ID NO:30 as shown in WO 96/40935, by a modification resulting in that the coding sequence has at least one codon coding for an amino acid residue that is not encoded by SEQ ID NO:30.

**4.** A nucleic acid fragment according to any of claims 1-3 where the nucleotide sequence coding for hexose oxidase is expressible in a host organism at a level which, relative to the level at which the non-modified nucleotide sequence is expressed in said host organism under substantially identical conditions, is at least 100% higher, including a nucleic acid fragment where the nucleotide sequence coding for hexose oxidase is extended by at least one codon at either end whereby, when the thus extended coding sequence is expressed, a C-terminally and/or N-terminally extended hexose oxidase is obtained.

**5.** A method of preparing a nucleic acid fragment comprising a modified nucleotide sequence coding for a hexose oxidase, the method comprising the step of

(i) selecting a source organism naturally producing a hexose oxidase,

(ii) isolating from said source organism a nucleic acid fragment comprising the nucleotide sequence coding for said naturally produced hexose oxidase,

(iii) selecting a host organism and determining for said host organism the frequency of nucleotide triplets (codons) coding for individual amino acids (codon usage), and

(iv) modifying the isolated nucleotide sequence by substituting at least one codon coding for an amino acid with a different codon coding for the same amino acid including such different codon that is used naturally by said host organism at a higher frequency than the codon being substituted, and

(v) selecting a thus modified nucleotide sequence that, when expressed in the selected host organism, is expressible in the selected host organism at a level which, relative to the level at which the non-modified nucleotide sequence is expressed in said host organism under substantially identical conditions, is at least 10% higher, including a method wherein the nucleic acid fragment comprising the nucleotide sequence coding for said naturally produced hexose oxidase is isolated from an algal species such as a species selected from the group consisting of *Chondrus crispus*, *Iridophycus flaccidum* and *Euthora cristata*, e.g. a method wherein the nucleotide sequence coding for said naturally produced hexose oxidase is SEQ ID NO:30 as shown in WO 96/40935.

**6.** A method according to claim 5 wherein the nucleotide sequence coding for hexose oxidase is modified to comprise at least one codon that codes for an amino acid residue which does not occur in the naturally produced hexose oxidase or wherein the resulting nucleic acid fragment comprises at least part of the coding sequence of SEQ ID NO:30 as shown herein, including a method wherein the resulting nucleic acid fragment comprises a coding sequence that is derived from the coding sequence of SEQ ID NO:30 by a modifaction that results in at least one codon coding for an amino acid residue that is not encoded by SEQ ID NO:30.

**7.** A method according to claim 5 wherein the resulting nucleic acid fragment comprises a coding sequence that is expressible in a host organism at a level which, relative to the level at which the non-modified nucleotide sequence is expressed in said host organism under substantially identical conditions, is at least 100% higher, including a method where the nucleic acid fragment coding for hexose oxidase is extended by at least one codon at either end whereby, when the thus extended coding sequence is expressed, a C-terminally and/or N-terminally extended hexose oxidase is obtained.

**8.** An expression vector that comprises the nucleic acid fragment according to any of claims 1-4 such as an expression vector wherein the nucleic acid fragment comprises, upstream of the translation initiation codon of the modified nucleotide sequence coding for hexose oxidase, a nucleotide sequence selected from the group consisting of TTATTCGAAGC and GGATCCAAACC and an expression vector that is selected from the group consisting of pUPO340 (DSM 12534), pUPO346 (DSM 12530) and pUPO349 (DSM 12531).

**9.** A host organism that is transformed with an expression vector according to claim 8, including a host organism that is selected from the group consisting of a bacterial species, a fungal species e.g. a yeast species including *Pichia pastoris* and a *Saccharomyces* species, a plant cell and an animal cell.

**10.** A host organism according to claim 9 that comprises a DNA sequence coding for a secretion signal that permits the hexose oxidase to be translocated across the host organism cell membrane or a host organism where the nucleotide sequence coding for hexose oxidase is under the control of a regulatory nucleotide sequence not naturally associated with the coding sequence.

**11.** A method of producing hexose oxidase comprising the steps of

(i) cultivating the host organism according to any of claims 9-10 in an appropriate medium under conditions where the nucleotide sequence coding for the hexose oxidase is expressed, and

(ii) harvesting the hexose oxidase, including a method wherein the amount of hexose oxidase that is expressed is at least 250 mg per liter of the cell culture volume after cultivation of the host organism such as at least 500 mg per liter, the method optionally comprising subjecting the harvested hexose oxidase to at least one further purification step.

**12.** A method of producing a polypeptide having hexose oxidase activity, comprising isolating a DNA fragment comprising a sequence coding for the polypeptide, transforming a host organism with said coding sequence, cultivating the thus transformed host organism under conditions leading to expression of the hexose oxidase active polypeptide and recovering the polypeptide from the cultivation medium, the polypeptide being expressed at an amount that is at least 100 mg per liter of the culture medium, preferably at least 250 mg per liter, including at least 500 mg per liter, the host organism preferably being a host organism of any of claims 9-10.

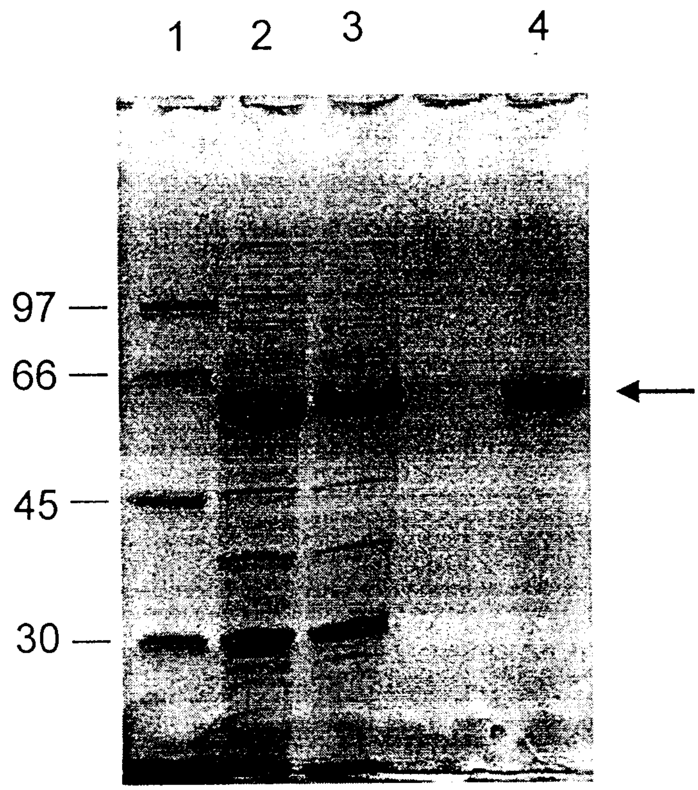


Fig. 1

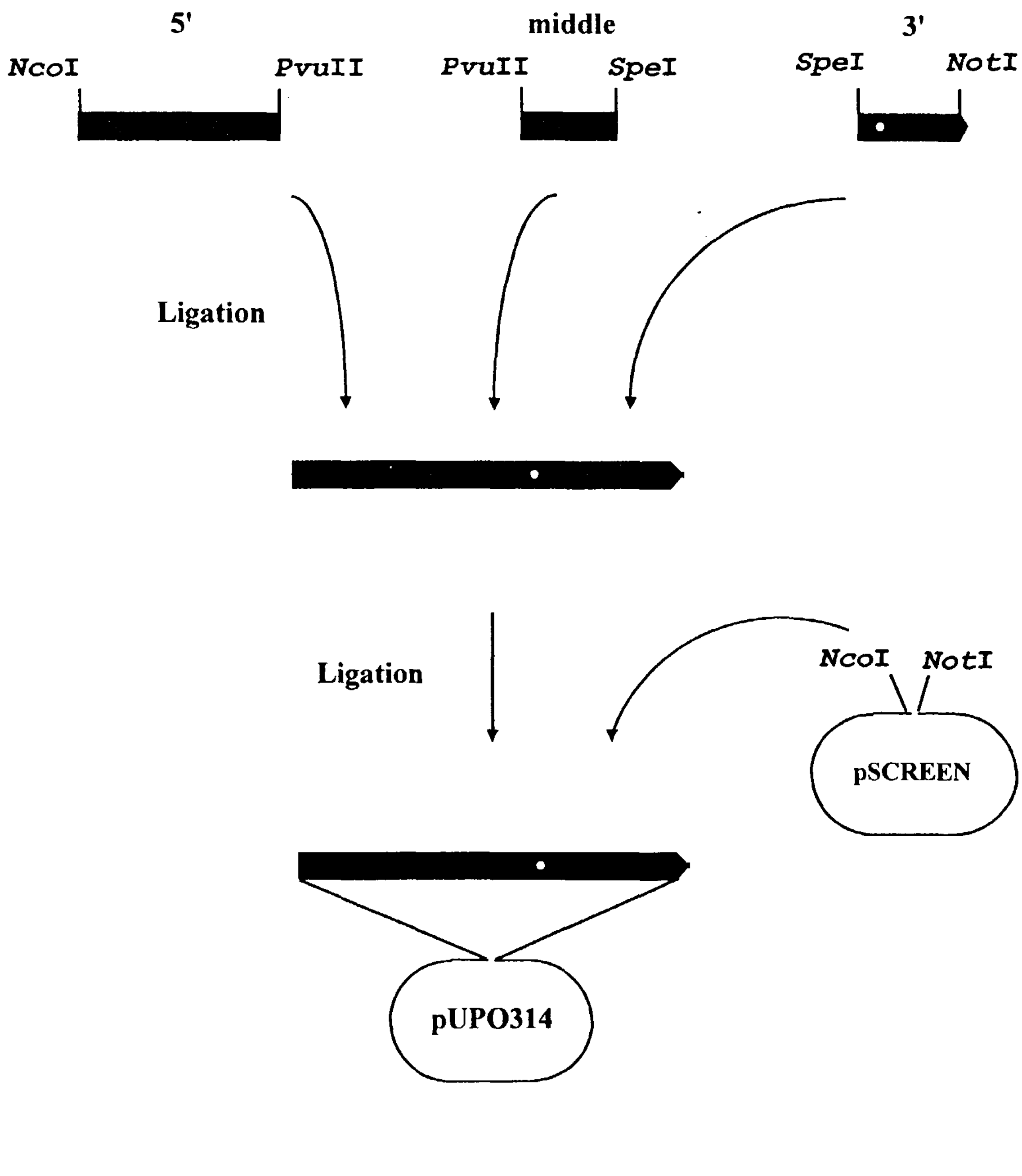
5'
middle
3'
NcoI
PvuII
PvuII
SpeI
SpeI
NotI
Ligation
Ligation
NcoI
NotI
pSCREEN
pUPO314

Fig. 2

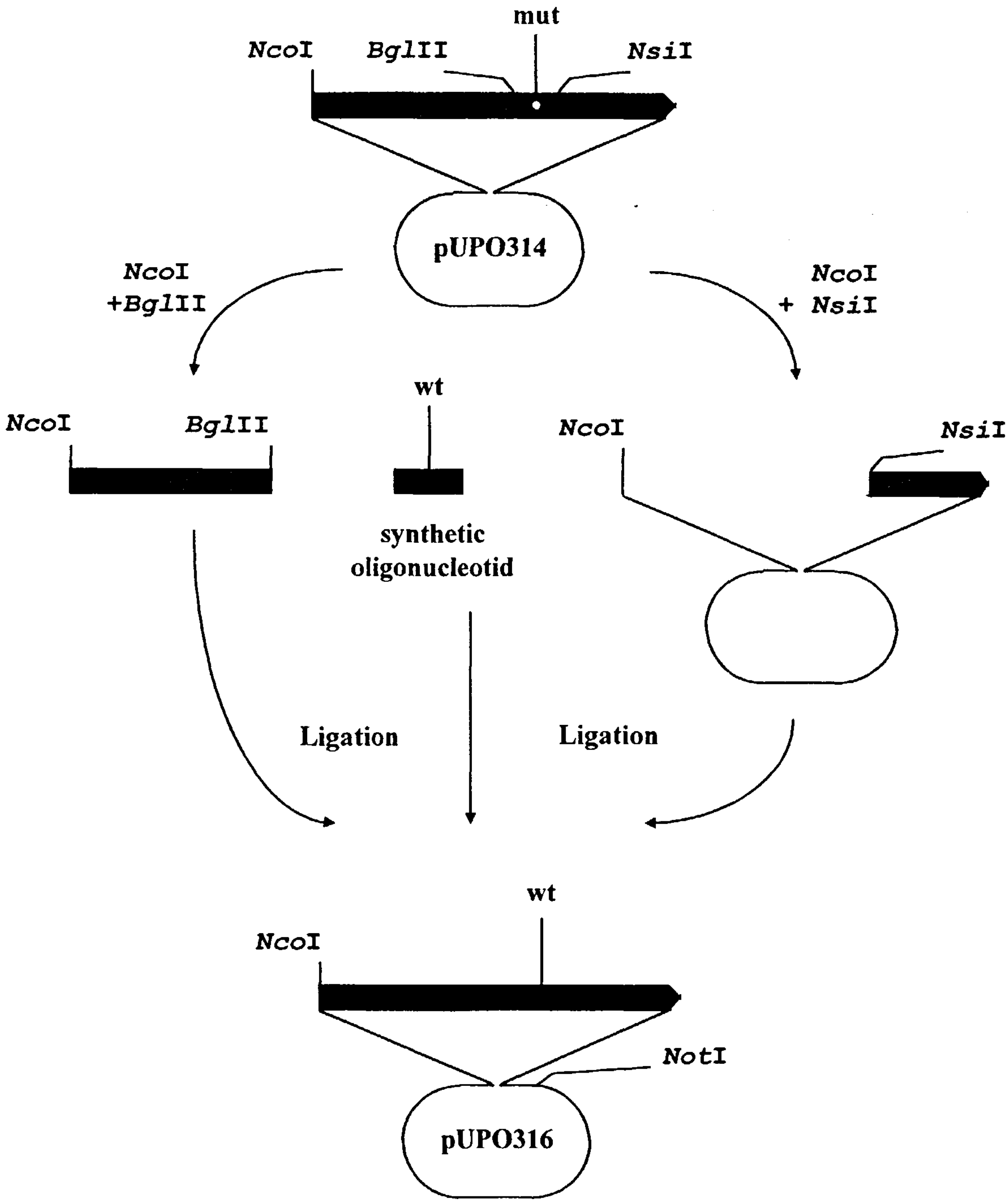
mut
NcoI
BglII
NsiI
pUPO314
NcoI
+BglII
NcoI
+ NsiI
wt
NcoI
BglII
NcoI
NsiI
synthetic
oligonucleotid
Ligation
Ligation
wt
NcoI
NotI
pUPO316

Fig. 3

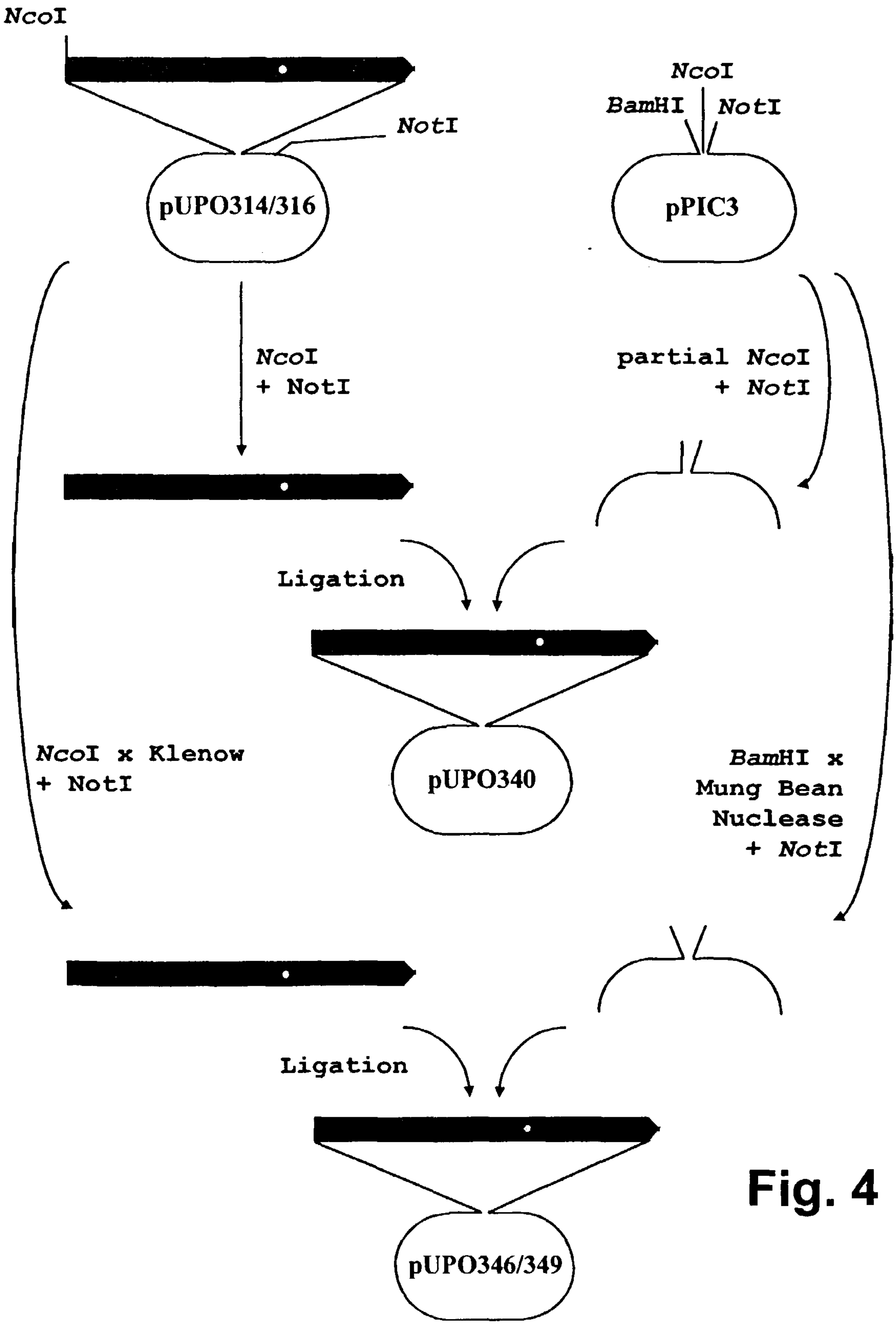
NcoI
NotI
pUPO314/316
NcoI
BamHI
NotI
pPIC3
NcoI
+ NotI
partial NcoI
+ NotI
Ligation
pUPO340
NcoI x Klenow
+ NotI
BamHI x
Mung Bean
Nuclease
+ NotI
Ligation
pUPO346/349

Fig. 4

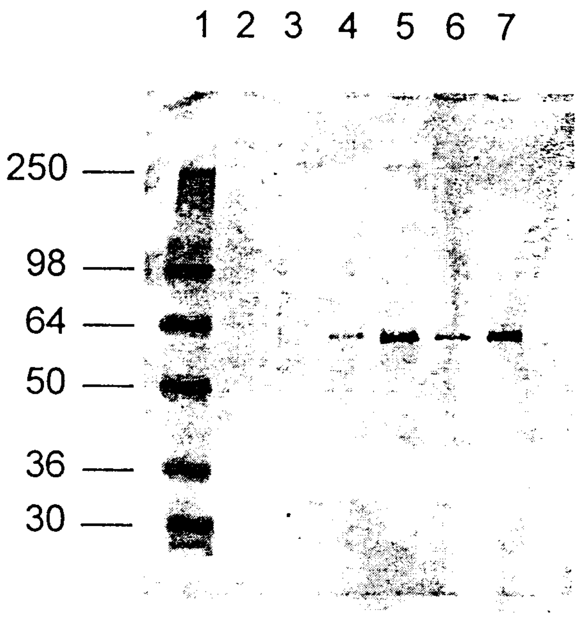


Fig. 5

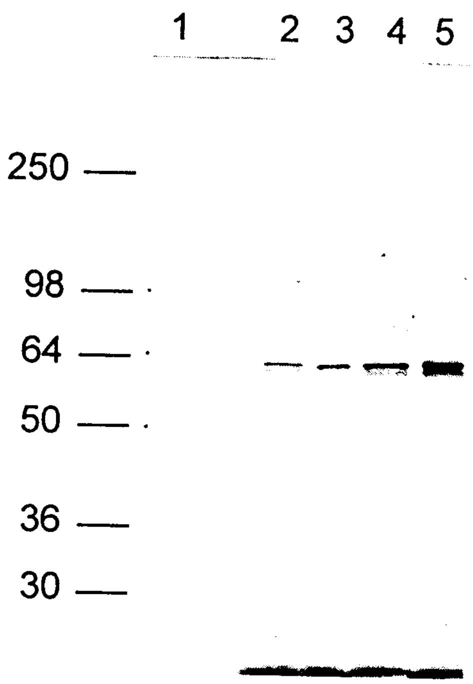


Fig. 6

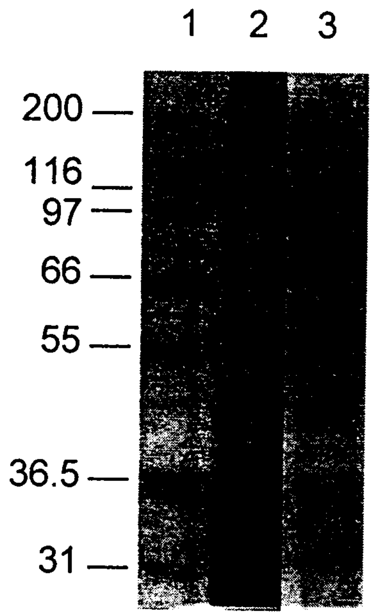


Fig. 7

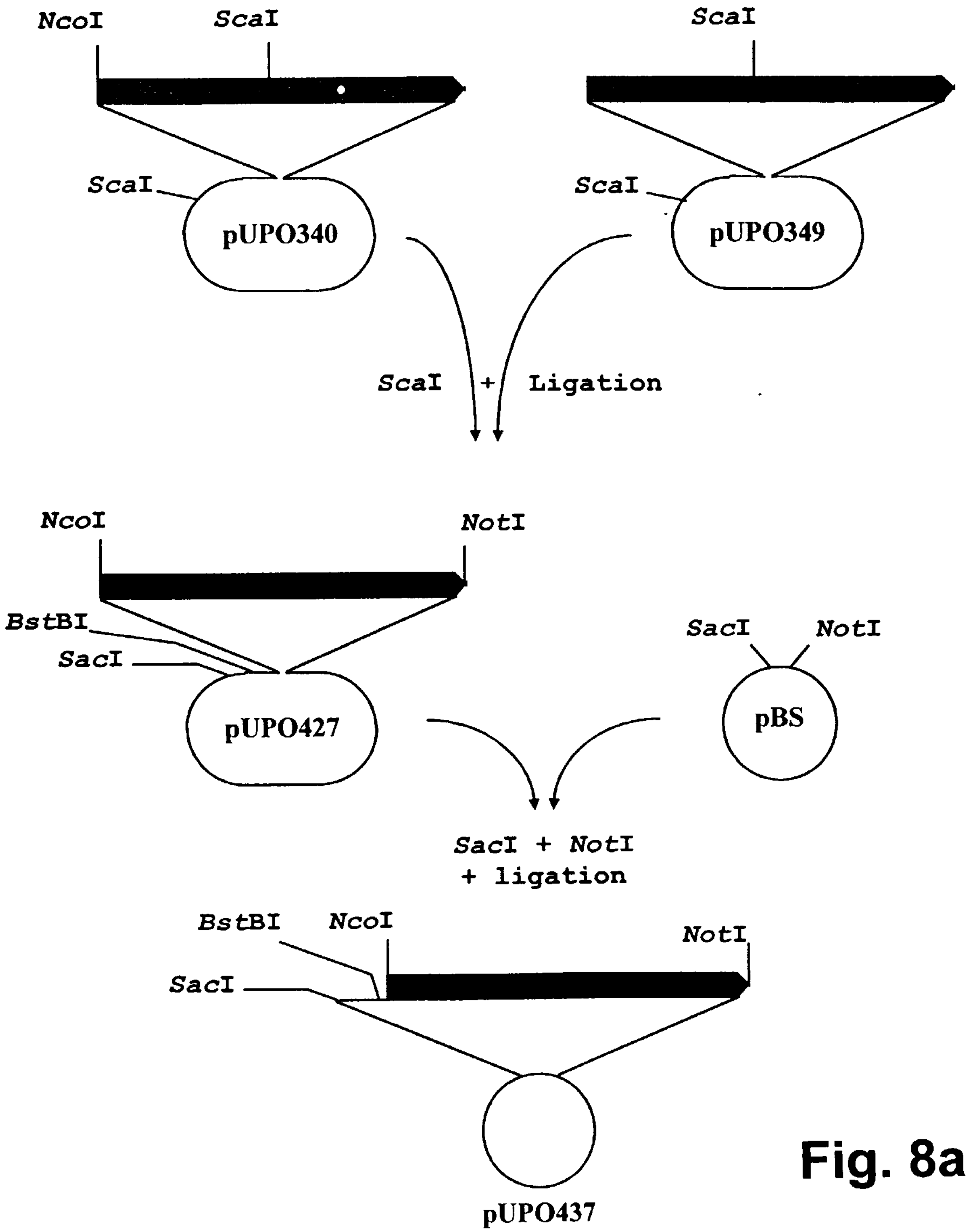
NcoI
ScaI
ScaI
ScaI
ScaI
pUPO340
pUPO349
ScaI + Ligation
NcoI
NotI
BstBI
SacI
pUPO427
SacI
NotI
pBS
SacI + NotI
+ ligation
BstBI
NcoI
NotI
SacI
pUPO437

Fig. 8a

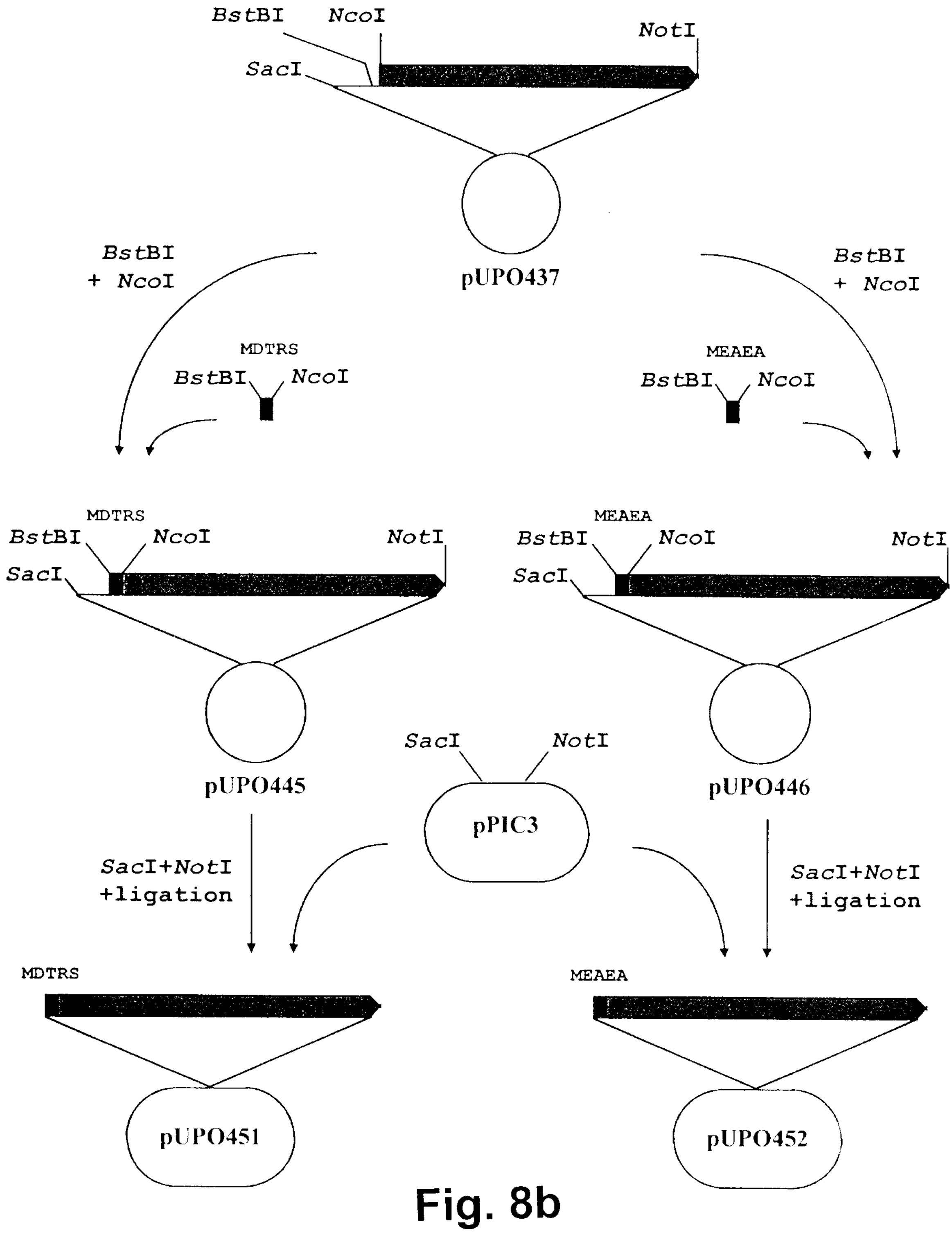
BstBI
NcoI
NotI
SacI
pUPO437
BstBI
+ NcoI
BstBI
+ NcoI
MDTRS
BstBI
NcoI
MEAEA
BstBI
NcoI
MDTRS
BstBI
NcoI
NotI
SacI
MEAEA
BstBI
NcoI
NotI
SacI
pUPO445
SacI
NotI
pPIC3
pUPO446
SacI+NotI
+ligation
SacI+NotI
+ligation
MDTRS
MEAEA
pUPO451
pUPO452

Fig. 8b

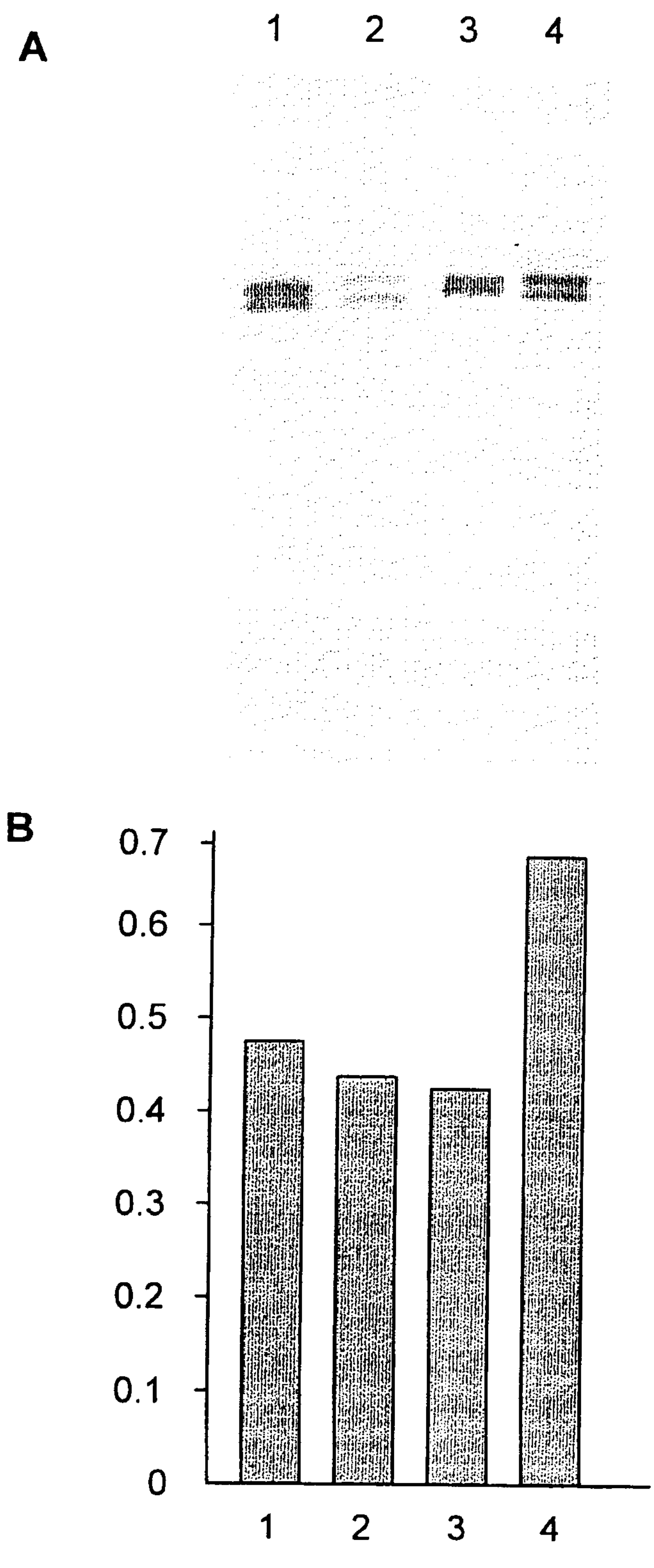


**Fig. 9**

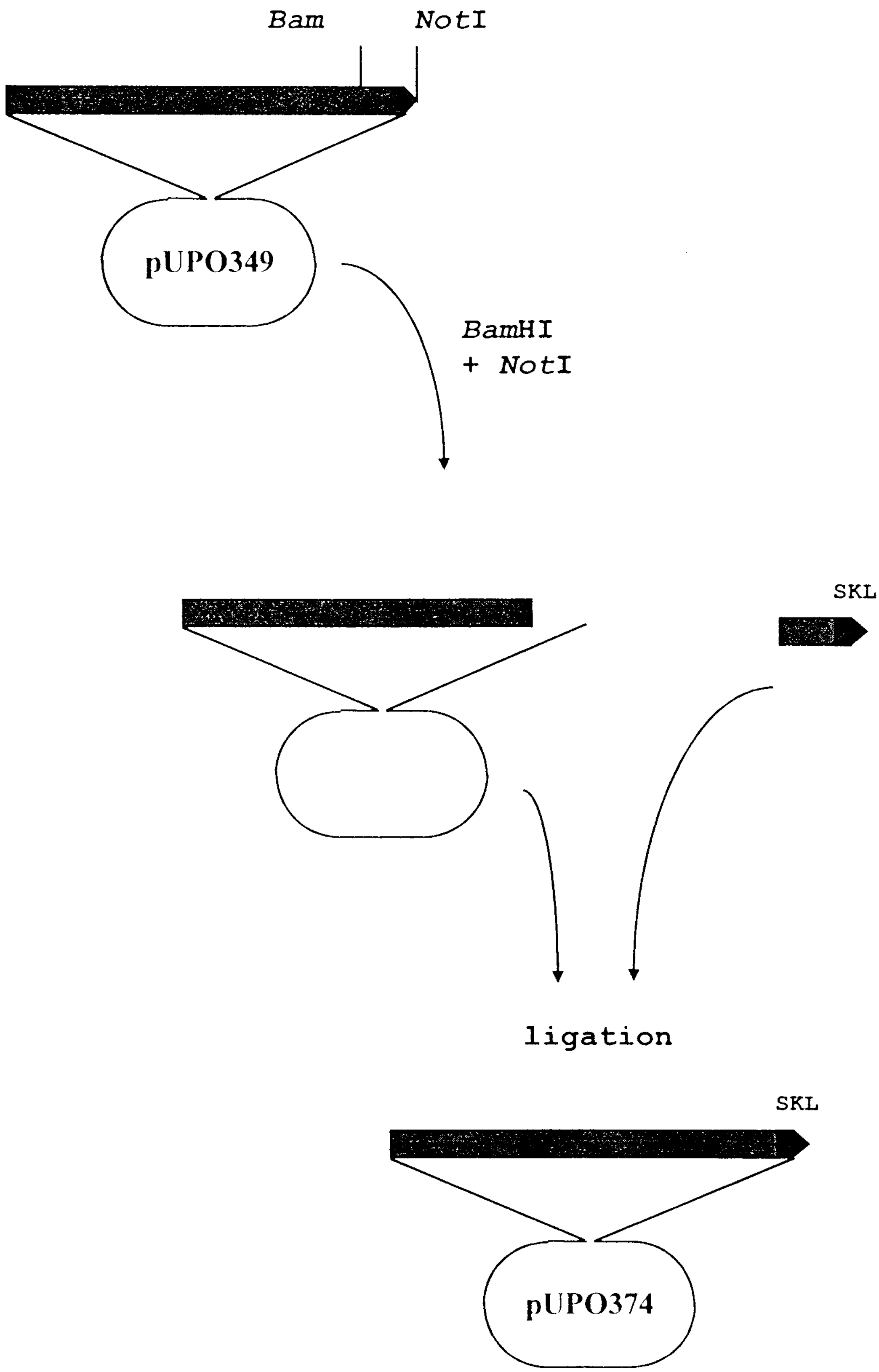


Fig. 10